# EUROPEAN PATENT SPECIFICATION

(11) **EP 1 904 469 B1**
(45) Date of publication and mention of the grant of the patent: **21.09.2011**
(21) Application number: 06762514.5
(22) Date of filing: 10.07.2006
(51) Int. Cl.: C07D 307/32, C07C 47/20

(54) **NEW PYROCATECHIN DERIVATIVES**
NEUE PYROCATECHINDERIVATE
NOUVEAUX DÉRIVÉS DE PYROCATÉCHINE

(30) Priority: 11.07.2005 EP 05014950
(43) Date of publication of application: 02.04.2008
(73) Proprietor: Novartis AG, 4056 Basel (CH)
(72) Inventor: SEDELMEIER, Gottfried, 79227 Schallstadt (DE); NEBEL, Kurt, CH-4146 Hochwald (CH); VEENSTRA, Siem, Jacob, 79540 Lörrach (DE); ZERGENYI, Janos, CH-4411 Seltisberg (CH)
(74) Representative: Woodcock-Bourne, Heather
(86) International application number: PCT/EP2006/006731
(87) International publication number: WO 2007/006532

(56) References cited:
- EP-A- 0 678 503
- WO-A-02/02508
- WO-A-02/08172
- BERLIN ET AL.: "Condensation of arenediols with phenylphosphonic dichloride. process of ring opening in bicyclic phosphonates leading to unsubstituted omicron-hydroxyaryl hydrogen phenylphosphates" TETRAHEDRON, vol. 20, no. 11, 1964, pages 2709-2716, XP002398639
- ARNOLDI ET AL.: "Synthesis and sweet taste of some 2-phenylbenzodioxanes" JOURNAL OF AGRICULTURAL AND FOOD CHEMISTRY, vol. 34, no. 2, 1986, pages 339-344, XP002398640
- VERTER H S ET AL: "SYNTHESIS OF 3-HYDROXY-4-METHOXYBUTYROPHENONE" JOURNAL OF CHEMICAL AND ENGINEERING DATA, AMERICAN CHEMICAL SOCIETY, US, vol. 9, no. 3, 1964, pages 403-404, XP000908986 ISSN: 0021-9568
- FORREST ET AL.: "Cleavage of the methylenedioxy ring in the Grignard reaction of 3,4-methylenedioxybenzonitrile" CANADIAN JOURNAL OF CHEMISTRY, vol. 52, no. 22, 1974, pages 3784-3786, XP008068870

## Description

The invention relates to new compounds of formula I 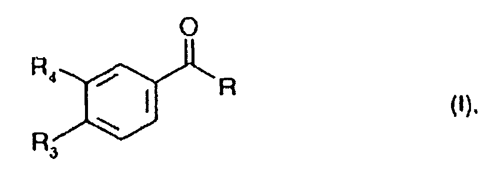 wherein
R is a group of formulae la, Ib, Ic or Id 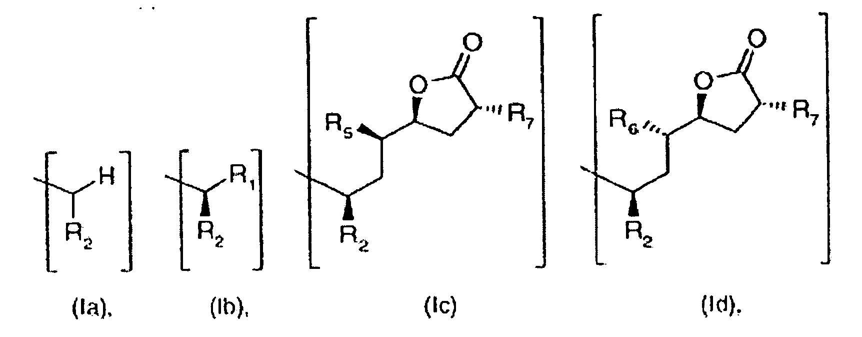 R₁ is 4-halogenbut-2-enyl ,
R₂ is C₁-C₇- alkyl or cycloalkyl,
R₃ is C₁-C₇- alkoxy,
R₄ is C₁-C₇- alkoxy - C₁-C₇- alkoxy, or where R is a group of formula (Ia), it is hydroxy, hydroxyl C₁-C₇- alkoxy or a group of formula Ie 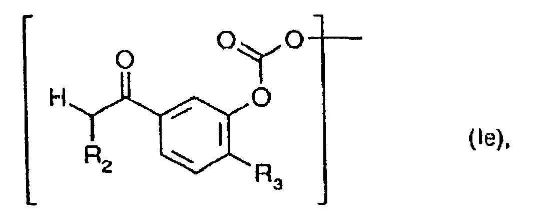 R₅ is reactive, esterified hydroxyl as defined in claim 1,
R₆ is azido and
R₇ is C₁-C₇- alkyl, C₁-C₇- alkenyl, cycloalkyl or aryl C₁-C₇- alkyl,
and their salts, a process for their preparation and their use as intermediates in the production of active ingredients for medicaments.

Halobut-2-enyl is, for example, 4-bromobut-2-enyl or 4-chlorobut-2-enyl, especially 4-bromobut-2-enyl.

Cycloalkyl has, for example, 3 to 8 , preferably 3 ring members, and signifies primarily cyclopropyl, or also cyclobutyl, cyclopentyl or cyclohexyl.

Reactive esterified hydroxy is halogen or C₁-C₇- alkanesulfonyloxy, halogen C₁-C₇- alkanesulfonyl, or benzenesulfonyloxy or naphthalenesulfonyloxy either unsubstituted or substituted by C₁-C₇- alkyl, halogen and/or nitro.

Hereinbefore and hereinafter, by C₁-C₇- radicals and compounds is understood, for example, those radicals and compounds having up to and including 7, preferably up to and including 4 carbon atoms (C-atoms).

Halogen is, for example, halogen with an atomic number from 19 to 35, such as chlorine or in particular bromine.

Halogen C₁₀-C₇- alkanesulfonyloxy is, for example, polyhalogen-C₁-C₄-alkanesulfonyloxy, such as trifluoromethanesulfonyloxy.

C₁-C₇- alkanesulfonyloxy is, for example C₁-C₄-alkanesulfonyloxy, such as methanesulfonyloxy, ethanesulfonyloxy, propanesulfonyloxy or butanesulfonyloxy.

C₁-C₇- alkenyl may be straight-chained or branched and is for example a corresponding C₂-C₄-alkenyl, such as allyl.

C₁-C₇- alkenesulfonyloxy is, for example C₂-C₄-alkenesulfonyloxy, such as ethenesulfonyloxy.

C₁-C₇- alkyl may be straight-chained or branched and is, for example, corresponding C₁-C₇-alkyl, especially C₁-C₄-alkyl, such as methyl, ethyl, propyl, isopropyl, butyl, isobutyl, secondary butyl, tertiary butyl or a pentyl, hexyl or heptyl group. Lower alkyl R₂ or R₅ is, for example, methyl or in particular branched C₃-C₇-alkyl, such as propyl.

C₁-C₇- alkoxy is, for example, C₁-C₇-alkoxy, preferably C₁-C₄-alkoxy, such as methoxy, ethoxy, propyloxy, isopropyloxy, butyloxy, isobutyloxy, secondary butyloxy, tertiary butyloxy, pentyloxy or a hexyloxy or heptyloxy group.

Hydroxy C₁-C₇- alkoxy is, for example, hydroxy-C₂-C₄-alkoxy, such as 2-hydroxyethoxy, 3-hydroxypropyloxy or 4-hydroxybutyloxy, especially 3-hydroxypropyloxy or 4-hydroxybutyloxy.

C₁-C₇- alkoxy C₁-C₇- alkoxy is, for example, C₁-C₄-alkoxy-C₂-C₄-alkoxy, such as 2-methoxy-, 2-ethoxy- or 2-propyloxyethoxy, 3-methoxy- or 3-ethoxypropyloxy or 4-methoxybutyloxy, especially 3-methoxypropyloxy or 4-methoxybutyloxy.

Phenyl C₁-C₇- alkyl is, for example, phenyl-C₁-C₄-alkyl, preferably 1-phenyl-C₁-C₄-alkyl, such as benzyl or 1-phenylethyl.

Salts are especially phenolate salts of compounds of formula I, wherein R₄ is hydroxy with appropriate bases, such as metal salts derived from metals of group Ia, Ib, IIa and IIb of the periodic table of elements, e.g. alkali metal salts, especially lithium, sodium or potassium salts, alkaline earth metal salts, for example magnesium or calcium salts, also zinc salts.

The invention was based on the problem of developing an improved method of obtaining compounds of formula II 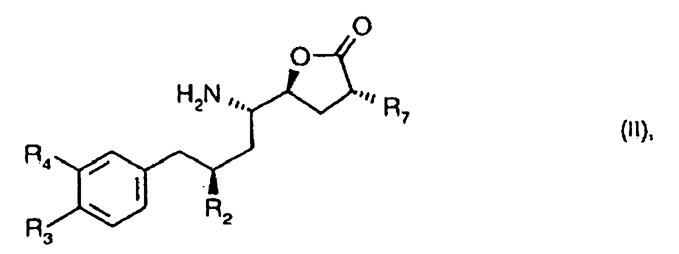 wherein R₂, R₃ and R₇ have the indicated significances, and R₄ signifies C₁-C₇- alkoxy lower alkoxy, which are valuable intermediates for the production of active ingredients for medicaments. For example, compounds of formula II according to EP-678503 are used as preferred intermediates for the production of compounds of formula 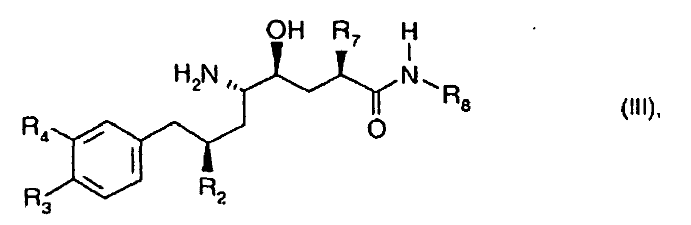 wherein R₂, R₃, R₄ and R₇ have the indicated significances, R₄ signifies C₁-C₇-alkoxy lower alkoxy, and R₈ signifies C₁-C₇-alkyl, cycloalkyl, optionally aliphatically esterified or etherified hydroxy C₁-C₇-alkyl, optionally N- C₁-C₇- alkanoylated, N-mono- or N,N-di C₁-C₇- alkylated amino C₁-C₇-alkyl, or amino C₁-C₇-alkyl N,N-disubstituted by C₁-C₇-alkylene, hydroxy-, C₁-C₇-alkoxy- or C₁-C₇-alkanoyloxy C₁-C₇-alkylene, optionally N'- C₁-C₇- alkanoylated or N'- C₁-C₇-alkylated aza C₁-C₇-alkylene, oxa C₁-C₇-alkylene or optionally S-oxidised thia C₁-C₇-alkylene; optionally esterified or amidated carboxy C₁-C₇-alkyl, optionally esterified or amidated dicarboxy C₁-C₇-alkyl, optionally esterified or amidated carboxy(hydroxy) C₁-C₇-alkyl, ooptionally esterified or amidated carboxycycloalkyl C₁-C₇-alkyl, cyano C₁-C₇-alkyl, C₁-C₇-alkanesulfonyl C₁-C₇-alkyl, optionally N-mono- or N,N-di- C₁-C₇- alkylated thiacarbamoyl C₁-C₇-alkyl, optionally N-mono- or N,N-di- C₁-C₇- alkylated sulfamoyl C₁-C₇-alkyl; or a heteroaryl radical which is bonded by a C-atom and is optionally hydrated and/or oxo-substituted; or a C₁-C₇-alkyl which is substituted by a heteroaryl radical which is bonded by a C-atom and is optionally hydrated and/or oxo-substituted, and their salts.

The intermediates of formula II are at present prepared in a four-step synthesis from 2- C₁-C₇- alkoxyphenols on the one hand and phenyl alaninol on the other hand, in accordance with the following scheme.

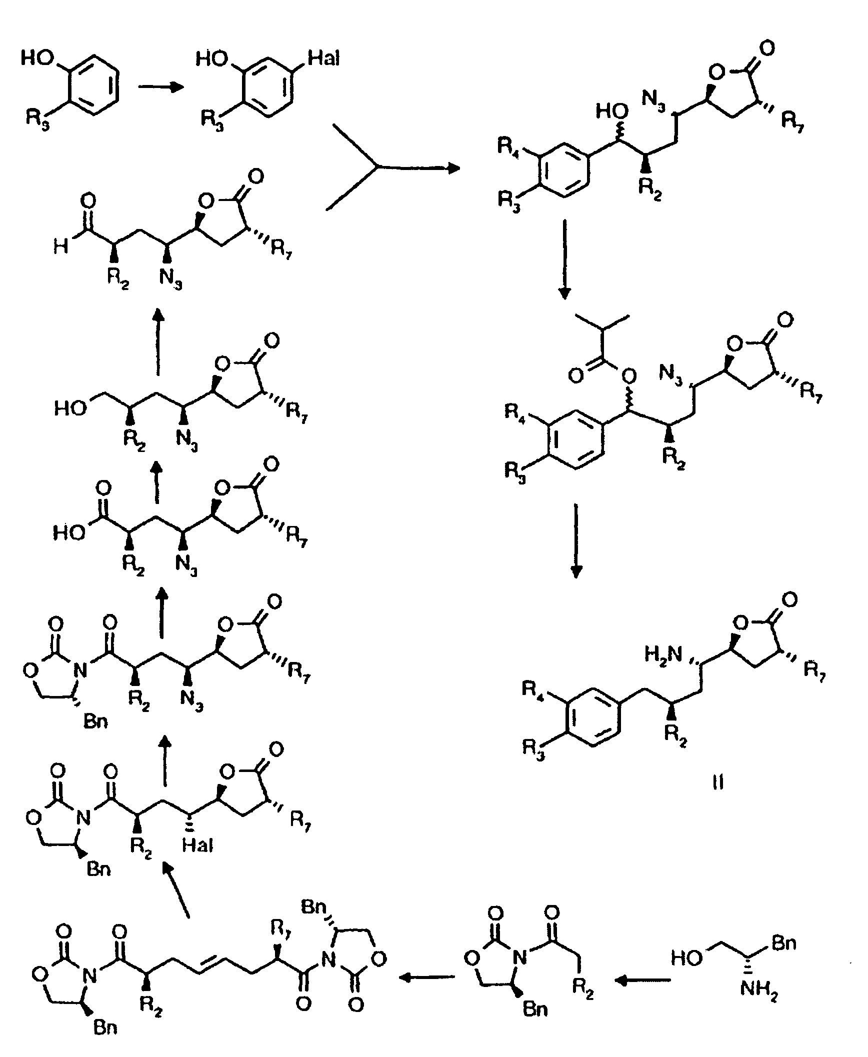

On a technical scale, the production of these starting materials and their further processing to the intermediates II is extremely complex, technical and, from a safety point of view, difficult to control, and expensive. There was therefore an urgent need for an improved and technically practicable alternative process for the production of compounds of formula II. Berlin et al., Tetrahedron, Vol. 20, No. 11, 1964, 2709-2716, discloses synthetic procedures for the condensation of arenediols with phenyl phosphonic dichloride.

Arnoldi et al., Journal of Agricultural and Food Chemistry, Vol. 34, No. 2, 1986, 339-344, discloses various 2-phenyl benzodioxanes.

Verther et al., Journal of Chemical Engineering Data, American Chemical Society, US, Vol. 9, No. 3, 1964, 403-404, discloses the synthesis of 3-hydroxy-4-methoxy butyrophenone.

Forrest et al. Canadian Journal of Chemistry, Vol. 52, No. 22, 1974, 3784-3786, discloses studies on the cleavage of the methylene dioxy ring of the Grignard reaction of 3,4-methylene dioxybenzonitrile.

WO 02/02508 and WO 02/08172 disclose pharmaceutically active compounds.

The solution to the problem according to the invention is based on the consideration that instead of halogenated, it is more advantageous to use acylated 2- C₁-C₇- alkoxyphenols, which already contain part of the side chain, and is based on the surprising discovery that the acylation of 2,2'-di C₁-C₇- alkoxy diphenylcarbonates takes place with high selectivity in p-position to the C₁-C₇- alkoxy groups. The process according to the invention uses methods which can be carried out more easily on a technical scale and are less problematic from an ecological and safety point of view than the known process.
Accordingly the present invention provides the subject matter of claim 1 to 6.
The solution, according to the invention, to the problem of producing the intermediates II is clarified by the following reaction scheme.

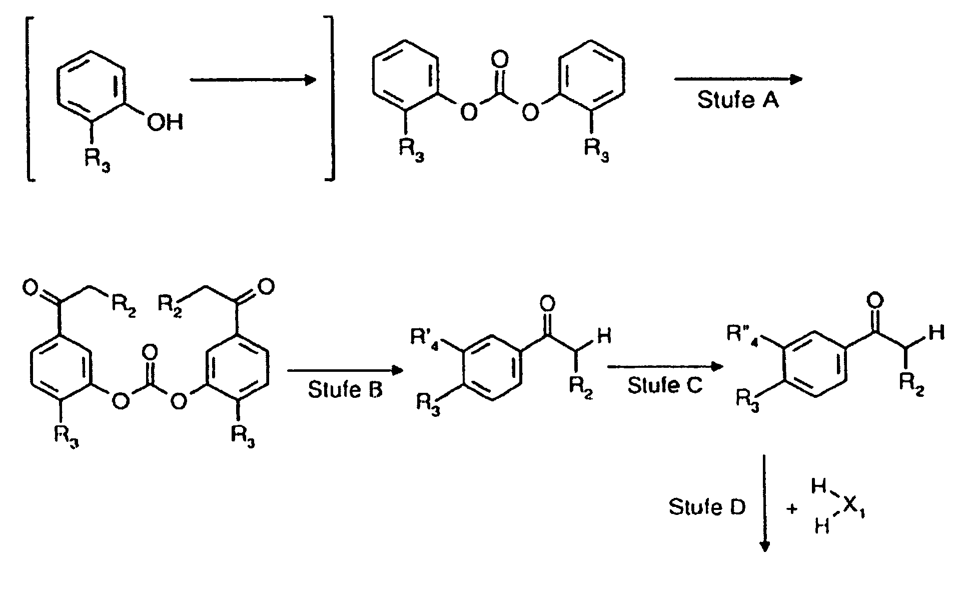

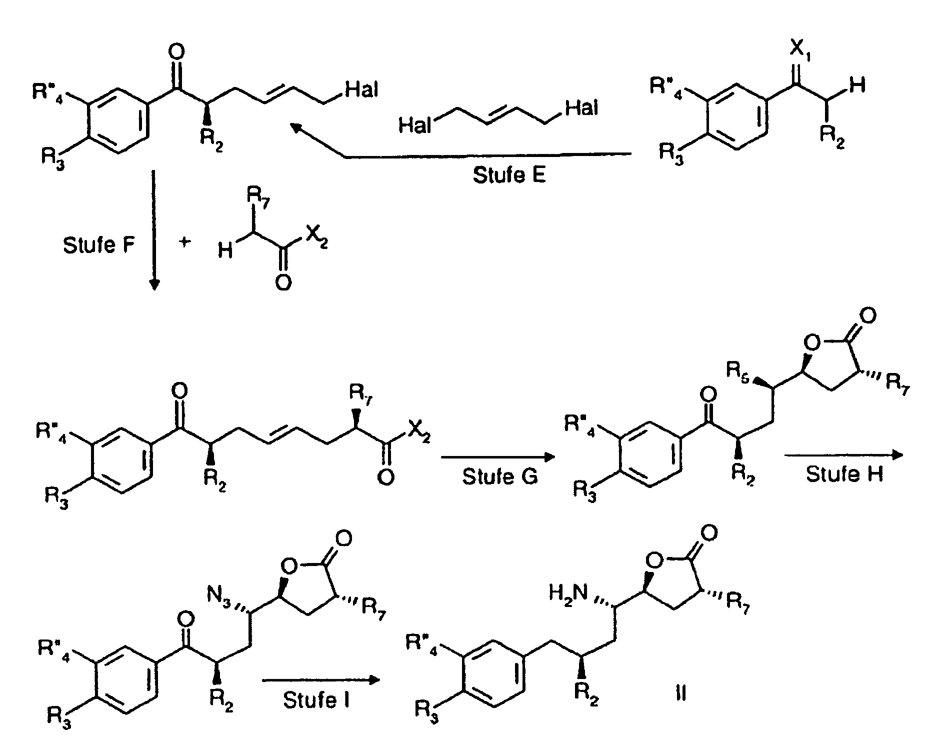

In a variation of this reaction sequence, steps E and F can also be exchanged by reacting the 1,4-dihalogen-but-2-ene in accordance with the reaction scheme 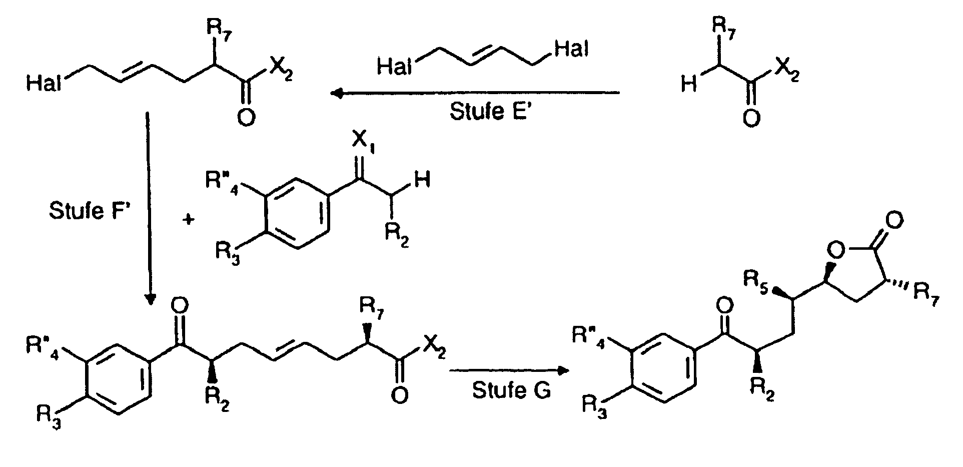 first of all with the chiral amide and then with the chiral ketimine which is obtainable according to steps D+E.

In the depicted reaction schemes, R'₄ signifies hydroxy C₁-C₇- alkoxy, R"₄ signifies C₁-C₇-alkoxy C₁-C₇- alkoxy, X₁ signifies the imino group derived from a chiral primary amine which is customary for the purpose of stereoselective synthesis, such as (S)-1-phenylethylamine or (S)-2-amino-1-methoxy-3-phenylpropane, and X₂ signifies a chiral amino, amido or urethane group which is customary for the purpose of stereoselective synthesis, or a chiral alcohol group. Compounds containing groups of this kind are, for example, pseudoephedrine, 4(S)-benzyl-2-oxo-oxazolidine, camphor sultam, borneol, intermediately-protected (S,S)- or (R,R)-2-aminophenylpropanediol, menthol, 8-phenylmenthol, pantolactone and the like.

In addition to the compounds of formula I prepared according to the invention, the invention also relates to steps A, B, C, D+E, F+G or F'+F'+G and H for their production, and the whole process, comprising these steps, for producing compounds of formula I, wherein R is a group of formulae Ic and Id, R₂ is C₁-C₇- alkyl or cycloalkyl, R₃ is C₁-C₇- alkoxy, R₄ is C₁-C₇- alkoxy C₁-C₇- alkoxy, R₅ is reactive esterified hydroxyl as defined in claim 1, especially halogen, R₆ is azido and R₇ is C₁-C₇- alkyl, C₁-C₇- alkenyl, cycloalkyl or aryl C₁-C₇- alkyl.

The use of compounds of formula I for the production of intermediates of formula II, in order to produce active ingredients for medicaments, of formula III, described in EP-678503, is a further object of the present invention.

The invention relates primarily to compounds of formula I, wherein
R illustrates a group of formulae la, Ib, Ic or Id,
R₁ is 4-halogenbut-2-enyl ,
R₂ is C₁-C₇- alkyl or 3- to 8-membered cycloalkyl,
R₃ is C₁-C₇- alkoxy,
R₄ illustrates C₁-C₇- alkoxy C₁-C₇- alkoxy, or where R is a group of formula (Ia), it is hydroxy, hydroxy C₁-C₇- alkoxy or a group of formula Ie.
R₅ is halogen or sulfonyloxy, such as C₁-C₇- alkane sulfonyloxy, halogen C₁-C₇- alkane sulfonyl, C₁-C₇- alkane sulfonyloxy; or benzene sulfonyloxy or naphthalene sulfonyloxy which is unsubstituted or substituted by C₁-C₇- alkyl, halogen and/or nitro.
R₆ is azido and
R₇ is C₁-C₇- alkyl, C₁-C₇- alkenyl, 3- to 8-membered cycloalkyl, or phenyl C₁-C₇- alkyl which is unsubstituted or substituted in the phenyl moiety by C₁-C₇- alkyl, C₁-C₇- alkoxy, halogen and/or nitro,
and their salts, a process for their preparation and their use as intermediates in the production of active ingredients for medicaments.

The invention preferably relates to compounds of formula I, wherein
R illustrates a group of formulae Ia, Ib, Ic or Id,
R₁ is 4-halogenbut-2-enyl ,
R₂ is branched C₃-C₇-alkyl, such as propyl,
R₃ is C₁-C₄-alkoxy, such as methoxy,
R₄ is C₁-C₄-alkoxy-C₂-C₄-alkoxy, such as 3-methoxypropyloxy, or where R is a group of formula (Ia), it is hydroxy, hydroxy-C₂-C₄-alkoxy, such as 3-hydroxypropyloxy, or a group of formula Ie,
R₅ is halogen of atomic numbers up to and including 35, such as bromine,
R₆ is azido or amino and
R₇ is branched C₃-C₇-alkyl, such as propyl,
and their salts, a process for their preparation and their use as intermediates in the production of active ingredients for medicaments.

The invention relates specifically to the compounds of formula I and their salts named in the examples, processes for their preparation, and their use as intermediates for producing active ingredients for medicaments.

The process according to the invention for the preparation of the compounds of formula I is characterised in that
a) in order to produce compounds of formula I, wherein R is a group of formula Ia, R₂ is C₁-C₇- alkyl or cycloalkyl, R₃ is C₁-C₇- alkoxy and R₄ is a group of formula Ie,
   a compound of formula IV 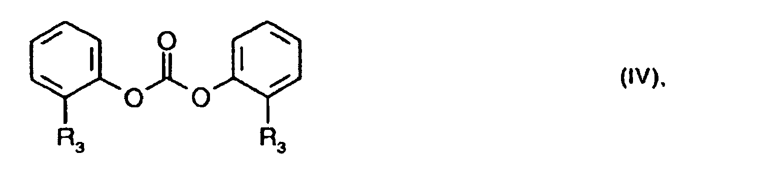 wherein R₃ has the above significance, is condensed with a compound of formula V 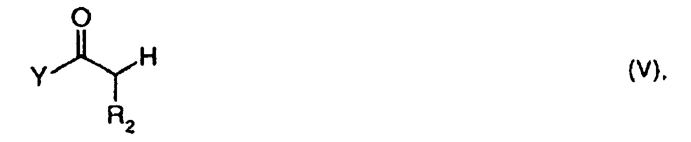 wherein R₂ has the above significance and Y is a reactive esterified hydroxy group,
b) in order to produce a compound of formula I, wherein R is a group of formula la, R₂ is C₁-C₇- alkyl or cycloalkyl, R₃ is C₁-C₇- alkoxy and R₄ is hydroxy, a compound of formula VI, 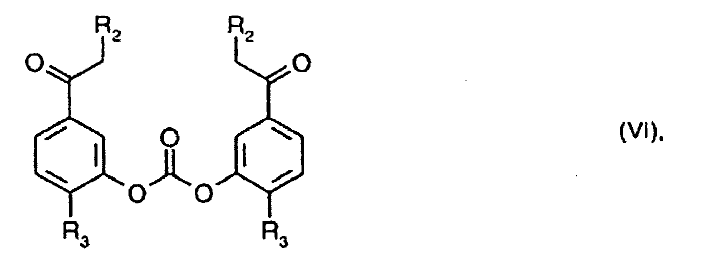 wherein R₂ and R₃ have the above significances, undergoes solvolysis to form the corresponding compounds of formula I, wherein R₄ is hydroxy,
c) in order to produce a compound of formula I, wherein R is a group of formula la, R₂ is C₁-C₇- alkyl or cycloalkyl, R₃ is C₁-C₇- alkoxy and R₄ is hydroxy C₁-C₇- alkoxy R'₄, in a compound of formula VII, 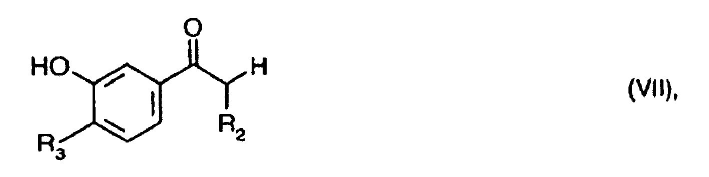 wherein R₂ and R₃ have the above significances, the hydroxy group is converted into hydroxy C₁-C₇- alkoxy R'₄,
d) in order to produce a compound of formula I, wherein R is a group of formula la, R₂ is C₁-C₇- alkyl or cycloalkyl, R₃ is C₁-C₇- alkoxy and R₄ is C₁-C₇- alkoxy C₁-C₇- alkoxy R"₄, in a compound of formula VIII, 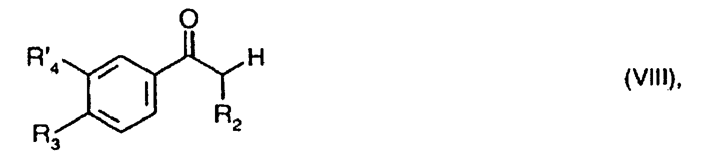 wherein R₂ and R₃ have the above significances and R'₄ is hydroxy C₁-C₇- alkoxy, the hydroxy C₁-C₇- alkoxy group is converted into C₁-C₇- alkoxy C₁-C₇- alkoxy R"₄,
e) in order to produce compounds of formula I, wherein R is a group of formula Ib, R₁ is 4-halogen-but-2-enyl, R₂ is C₁-C₇- alkyl or cycloalkyl, R₃ is C₁-C₇- alkoxy and R₄ is C₁-C₇-alkoxy C₁-C₇- alkoxy R"₄, a compound of formula IX 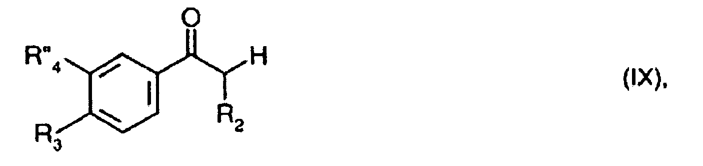 wherein R₂, R₃ and R"₄ have the above significances, is reacted first of all with a chiral primary amine which is customary for the purpose of stereoselective synthesis, and then with a 1,4-dihalogen-but-2-ene,
f) in order to produce compounds of formula I, wherein R is a group of formula Ic, R₂ is C₁-C₇- alkyl or cycloalkyl, R₃ is C₁-C₇- alkoxy, R₄ is C₁-C₇- alkoxy C₁-C₇- alkoxy R"₄, R₅ is reactive esterified hydroxyl as defined in claim 1 and R₇ is C₁-C₇- alkyl, C₁-C₇- alkenyl, cycloalkyl or aryl C₁-C₇- alkyl,
   a compound of formula X, 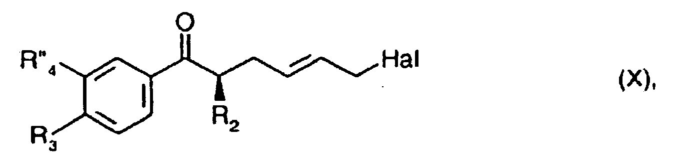 wherein R₂, R₃ and R"₄ have the above significances, and Hal is halogen, is reacted with a compound of formula XI 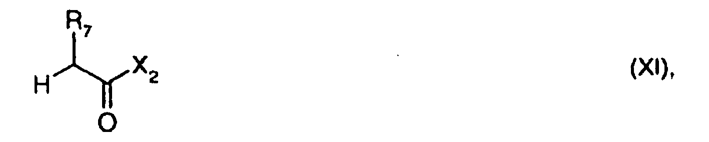 in which R₇ has the indicated significance and X₂ is a chiral amino, amido or urethane group which is customary for the purpose of stereoselective synthesis, or is a chiral alcohol group; or a 1,4-dihalogen-but-2-ene is reacted firstly with a compound of formula XI and then with the reaction product from the reaction of a compound of formula IX 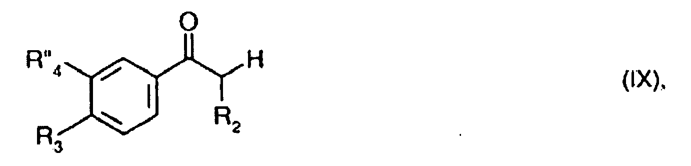 with a chiral primary amine of formula XII, which is customary for the purpose of stereoselective synthesis, 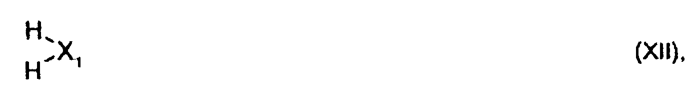 and the reaction product of formula XIII 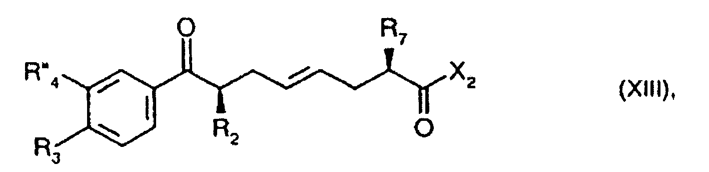 is condensed intramolecularly with lactonisation to form the corresponding compounds of formula I, wherein R is a group of formula Ic and R₅ is halogen, and if desired, halogen R₅ is converted stereoselectively into another reactive etherified hydroxy group R₅, and/or
g) in order to produce compounds of formula I, wherein R is a group of formula Id, R₂ is C₁-C₇- alkyl or cycloalkyl, R₃ is C₁-C₇- alkoxy, R₄ is C₁-C₇- alkoxy C₁-C₇- alkoxy R"₄, R₆ is azido and R₇ is C₁-C₇- alkyl, C₁-C₇- alkenyl, cycloalkyl or aryl C₁-C₇- alkyl, in a compound of formula XIV 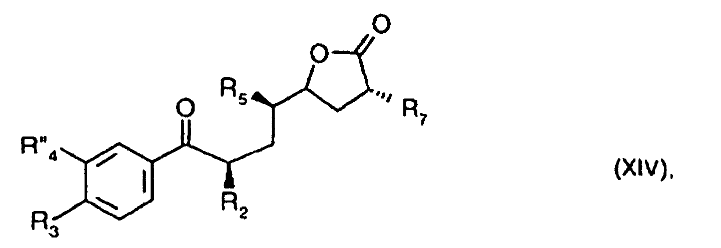 wherein R₂, R₃, R"₄ and R₇ have the above significances, and R₅ is reactive esterified hydroxyl as defined in claim 1, reactive esterified hydroxy R₅ is replaced stereospecifically by azido, and if desired, a free compound obtainable according to the method is converted into a salt, or a salt obtainable according to the method is converted into the free compound or into another salt.
   In starting substances of formula according to process variant a), reactive esterified hydroxy signifies, for example, hydroxy which is esterified with a hydrohalic acid or an organic sulfonic acid, such as halogen, especially chlorine, or optionally halogenated lower alkane sulfonyloxy, such as methane sulfonyloxy or trifluoromethane sulfonyloxy.
   The reaction of compounds of formulae IV and V is effected in the usual manner, preferably in the presence of an acidic condensation agent, for example a Lewis acid, especially aluminium trichloride, advantageously in an inert solvent, such as a halogenated hydrocarbon, such as methylene chloride, if necessary whilst cooling, preferably in a temperature range from ca. 5°C to ca. 30°C, for example at room temperature.
   The solvolysis of compounds of formula VI according to process variant b) is effected by conventional solvolysis processes, preferably under the conditions of a basic hydrolysis, for example by treatment with sodium hydroxide in an aqueous lower alkanol, such as methanol/water mixtures, advantageously with heating, preferably in a temperature range from ca. 55°C to ca. 90°C, for example at boiling temperature.
   The conversion of the hydroxy group R₄ into hydroxy C₁-C₇- alkoxy according to process variant c) may take place in conventional manner, for example by a reaction with a reactive monoester, such as a hydrohalic acid ester or sulfonic acid ester of a C₁-C₇- alkanediol, such as a ω-hydroxy C₁-C₇- alkyl halide, if necessary in the presence of a basic condensation agent, such as an alkali metal or alkaline earth metal hydroxide or an alkali metal carbonate, advantageously in a solvent which is inert towards the reaction components, if necessary with heating, for example in a temperature range of ca. 60°C to ca. 100°C, preferably by reacting with a ω-hydroxy C₁-C₇- alkyl halide in the presence of potassium carbonate in boiling acetonitrile.
   Conversion of the hydroxy C₁-C₇- alkoxy group R'₄ into C₁-C₇- alkoxy C₁-C₇- alkoxy R"₄ according to process variant d) may take place in conventional manner, for example by a reaction with a reactive ester of a C₁-C₇- alkanol, such as corresponding halides, sulfates or optionally halogenated C₁-C₇- alkane sulfonates, such as methane sulfonates or trifluoromethane sulfonates, if necessary in the presence of a basic condensation agent, such as an alkali metal or alkaline earth metal hydroxide, advantageously in a solvent which is inert towards the reaction components, if necessary with heating, for example in a temperature range of ca. 30°C to ca. 60°C, preferably by reacting with a di-lower alkyl sulfate in the presence of potassium hydroxide in toluene at ca. 40°C.
   Suitable amines for the reaction of compounds of formula IX according to process variant e) are chiral primary amines which are customary for the purpose of stereoselective synthesis, such as (S)-1-phenylethylamine or (S)-2-amino-1-methoxy-3-phenylpropane. The reaction of compounds of formula IX firstly with a chiral primary amine and then with a 1,4-dihalobut-2-ene is preferably carried out in a one-pot reaction without isolation of the intermediates in a solvent which is suitable for both steps, such as a solvent forming an azeotropic mixture with water, e.g. benzene or preferably toluene, if necessary adding one further or several further solvent(s), and in the second step adding a basic condensation agent, such as an alkali metal amide derivative, such as a N,N-bis(tri- C₁-C₇--alkylsilyl)-alkali metal amide, as well as a tertiary amide or lactam. Preferably, a solution of the compound of formula IX and the chiral amine is heated in toluene, firstly whilst distilling the reaction water, e.g. in a water separator, then concentrating to ca. two thirds to one half, then the basic condensation agent is added, for example a solution of a N,N-bis(tri- C₁-C₇--alkylsilyl)-alkali metal amide, preferably N,N-bis(trimethylsilyl)-lithium amide, in an ether-like solvent, such as tetrahydrofuran, and if necessary a tertiary amide or lactam is added, e.g. dimethylpropylene urea, then a solution of 1,4-dihalobut-2-ene is added whilst cooling, for example in a temperature range of ca. -10°C to ca. +10°C, and heated to room temperature, and working up is effected under slightly acidic conditions.
   In the reaction with compounds of formula X or 1,4-dihalobut-2-enes according to process variant f), suitable compounds of formula XI are, for example, those in which X₂ signifies a chiral amino, amido, urethane or alcohol group. Compounds containing groups of this kind are, for example, chiral amines, amides, urethanes and alcohols, which are customary for the purpose of stereoselective synthesis, for example pseudoephedrine, 4(S)-benzyl-2-oxo-oxazolidine, camphor sultam, borneol, intermediately-protected (S,S)- or (R,R)-2-aminophenylpropanediol, menthol, 8-phenylmenthol, pantolactone and the like.
   The reaction of compounds of formulae X and XI, likewise the reaction of 1,4-dihalo-but-2-enes and compounds of formula XI, preferably takes place in the presence of a basic condensation agent, for example an alkali metal amide derivative, such as a N,N-bis-(tri- C₁-C₇-alkylsilyl)-alkali metal amide, e.g. N,N-bis-(trimethylsilyl)-lithium amide, as well as a tertiary amide or lactam, advantageously in a solvent which is inert towards the reaction components, such as toluene, if necessary whilst cooling, e.g. in a temperature range from ca. -10°C to ca. +10°C, in an ether-like solvent, such as tetrahydrofuran, adding a solution of the 1,4-dihalo-but-2-ene whilst cooling, for example in a temperature range from ca. -10°C to ca. +10°C and working up under slightly acidic conditions, and heating to room temperature and working up under slightly acidic conditions.
   Suitable amines for the reaction of compounds of formula IX are, for example, chiral primary amines which are customary for the purpose of stereoselective synthesis, such as (S)-1-phenylethylamine or (S)-2-amino-1-methoxy-3-phenylpropane.
   The reaction of compounds of formula IX firstly with a chiral primary amine and then with an intermediate formed by reacting a 1,4-dihalobut-2-ene with a compound of formula XI, is preferably carried out in a one-pot reaction without isolation of the intermediates in a solvent which is suitable for both steps, such as a solvent forming an azeotropic mixture with water, e.g. benzene or preferably toluene, if necessary adding one further or several further solvent(s), and in the second step adding a basic condensation agent, such as an alkali metal amide derivative, such as a N,N-bis(tri- C₁-C₇-alkylsilyl)-alkali metal amide, as well as a tertiary amide or lactam. Preferably, a solution of the compound of formula IX and the chiral amine of formula XII is heated in toluene, firstly whilst distilling the reaction water, e.g. in a water separator, then concentrating to ca. two thirds to one half, then the basic condensation agent is added, for example a solution of a N,N-bis(tri- C₁-C₇-alkylsilyl)-alkali metal amide, preferably N,N-bis(trimethylsilyl)-lithium amide, in an ether-like solvent, such as tetrahydrofuran, and if necessary a tertiary amide or lactam is added, e.g. dimethylpropylene urea, then a solution of the intermediate formed by reacting a 1,4-dihalobut-2-ene with a compound of formula XI is added whilst cooling, for example in a temperature range of ca. -10° to ca. +10°C, heated to room temperature, and working up is effected under slightly acidic conditions.
   The intramolecular condensation of compounds of formula XIII with lactonisation is carried out, for example, by treatment with a halogenation agent, such as a N-halo-dicarboxylic acid imide, for example N-bromosuccinimide, advantageously in a two-phase solvent system, such as dichloromethane/water. The stereoselective exchange of halogen R₅ into another reactive etherified hydroxy groupas defined in claim 1 R₅ may be carried out in a conventional manner.
   The stereoselective replacement of halogen R₅ with azido according to process variant g) is effected in the usual manner, for example by a reaction with a metal or ammonium azide, for example with sodium azide, preferably in the presence of a quaternary nitrogen base, such as a quaternary aliphatic amine, e.g. N,N,N-tricapryl-N-methyl-ammonium chloride, if necessary whilst heating, e.g. in a temperature range from ca. 50°C to ca. 100°C, advantageously in a two-phase solvent system, such as toluene/water.
   According to the intended use of compounds of formula I prepared according to the invention, a further object of the invention is a process for the production of compounds of formula II 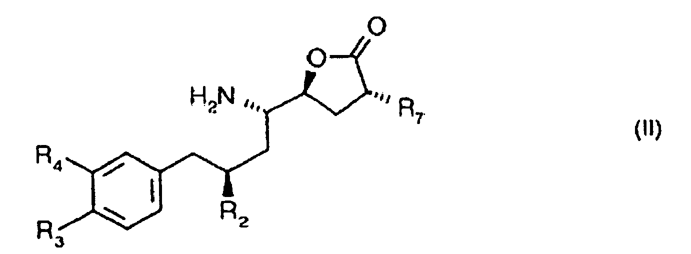 wherein R₂, R₃, R₄ and R₇ have the indicated significances, and their salts. This is characterised in that
h) a compound of formula I, wherein R is a group of formula Id, R₂ is C₁-C₇- alkyl or cycloalkyl, R₃ is C₁-C₇- alkoxy, R₄ is C₁-C₇- alkoxy C₁-C₇- alkoxy, R₆ is azido and R₇ is C₁-C₇-alkyl, C₁-C₇- alkenyl, cycloalkyl or aryl C₁-C₇- alkyl, the azido group R₆ is reduced to amino, and at the same time or subsequently, the benzoyl group is reduced to the corresponding benzyl group, for example by catalytic hydrogenation, for example in the presence of a palladium catalyst, such as palladium on carbon, at normal pressure and at room temperature, advantageously in a C₁-C₇- alkanol, such as ethanol, especially in a mixture with an aliphatic amino-alkanol, such as ethanolamine.

Salts of compounds of formula I and II, which are obtainable by the process, can be converted in known manner into the free compounds. For example, salts of compounds of formula II can be converted by treatment with a base, such as an alkali metal hydroxide, a metal carbonate or hydrogen carbonate, or ammonia, or with another salt-forming base mentioned initially, or with an acid, such as a mineral acid, e.g. hydrochloric acid, or with another salt-forming acid mentioned initially.

The following examples serve to illustrate the invention; the temperatures are indicated in degrees Celsius, and pressures in mbar.

### Example 1: Bis-(3-isovaleroyl-6-methoxy-phenyl)-carbonate

7.2 g of isovaleroyl chloride are dissolved in 30 ml of methylene chloride and mixed with 10.6 g of water-free aluminium chloride. Then, a total of 5.5 g of guaiacol carbonate is added in portions, whereby the reaction temperature is maintained at 20°, if necessary by cooling. The mixture is stirred for 1 hour at room temperature, poured onto ice, extracted with ethyl acetate, the organic phase separated, dried over sodium sulfate, concentrated by evaporation and crystallised from toluene. 86% of theory of the title compound is obtained, with a m.p. of 120-122°.

### Example 2: 1-(3-hydroxy-4-methoxy-phenyl)-3-methyl-butan-1-one

A solution of 7.0 g of bis-(3-isovaleroyl-6-methoxyphenyl)carbonate in 20 ml of methanol is mixed with 7 ml of 30% sodium hydroxide solution and heated at reflux whilst stirring for 1½ hours. The mixture is cooled to room temperature, mixed with 50 ml of water, slightly acidified with acetic acid, extracted with toluene and evaporated to dryness. The residue, which is oily at first, crystallises upon standing. 97% of theory of the title compound is obtained, with a m.p. of 51-53°.

### Example 3: 1-[3-(3-hydroxypropyloxy)-4-methoxy-phenyl]-3-methyl-butan-1-one

55 g of potassium carbonate and 30 ml of 3-chloro-1-propanol are added to a solution of 55 g of 1-(3-hydroxy-4-methoxy-phenyl)-3-methyl-butan-1-one in 250 ml of acetonitrile, and stirred at reflux for 20 hours. The suspension is filtered and concentrated by evaporation. 81 g of the title compound are obtained as the residue of evaporation. It crystallises upon cooling, m.p. 38-40°C.

### Example 4: 1-[4-methoxy 3-(3-methoxypropyloxy)-phenyl]-3-methyl-butan-1-one

40 g of powdered potassium hydroxide and 30 ml of dimethyl sulfate are added at 22-26°C to a solution of 50 g of 1-(3-(3-hydroxypropyloxy)-4-methoxy-phenyl]-3-methyl-butan-1-one in 150 ml of toluene. The mixture is stirred for 8 hours at 40°C. Then, 150 ml of water are added, and stirring continues for a further 12 hours at room temperature. The organic solvent is separated and concentrated, and the residue distilled under a high vacuum (220°C, 0.05 torr) 41 g is obtained as an oil. The total yield of examples 3 and 4 is 90% of theory.

### Example 5: trans-1-bromo-6-[4-methoxy-3-(3-methoxypropyloxy)-phenyl]-5-(S)-isopropyl-hex-2-en-6-one

A solution of 15.8 g of 1-[4-methoxy-3-(3-methoxypropyloxy)-phenyl]-3-methyl-butan-1-one and 9.3 g of (S)-(-)-2-amino-1-methoxy-3-phenylpropane in 100 ml of toluene is boiled whilst removing water until ca. 1 ml of water has been removed. Ca. 60 ml of toluene is distilled off. To the remaining solution of the imine of 1-[4-methoxy-3-(3-methoxypropyloxy)-phenyl]-3-methyl-butan-1-one are added at 0°C first of all 63 ml of a 1-molar solution of bis-trimethylsilyl-lithium amide in dry tetrahydrofuran and then 15.2 g of dimethylpropylene urea. The solution obtained is added dropwise at 0°C to a solution of 16 g of *trans* 1,4-dibromobut-2-ene in 26 ml of toluene. After stirring, the mixture is acidified with diluted hydrochloric acid, separated, and the organic solution concentrated. The crude product is purified by chromatography on a column of silica gel (eluant: hexanelethyl acetate 85:15).

### Example 6: trans-1-bromo-6-[4-(S)-benzyl-2-oxo-oxazolidin-3-yl]-(S)-isopropyl-hex-2-en-6-one

To a solution of 5.6 g of N-isovaleroyl-(S)-4-benzyl-oxazolidin-2-one in 12 ml of toluene are added, at 0°C, first of all 23.5 ml of a 1-molar solution of bis-trimethylsilyl-lithium amide in tetrahydrofuran, then 5.7 g of dimethylpropylene urea. The solution obtained is added dropwise at 0°C to a solution of 6 g of *trans* -1,4-dibromobut-2-ene in 10 ml of toluene. After stirring, the mixture is acidified with diluted hydrochloric acid, separated, and the organic solution concentrated. The crude product is purified by chromatography on a column of silica gel (eluant: hexane/ethyl acetate 85:15).

### Example 7: trans-1-[4-(S)-benzyl-2-oxo-oxazolidin-3-yl1-8-[4-methoxy-3-(3-methoxypropyloxy)-phenyl]-2(S)-7(S)-diisopropyl-oct-4-en-1,8-dione

A solution of 5.6 g of 1-[4-methoxy-3-(3-methoxypropyloxy)-phenyl]-3-methyl-butan-1-one and 3.3 g of (S)-(-)-2-amino-1-methoxy-3-phenylpropane in 30 ml of toluene is boiled whilst removing water until ca. 0.3 ml of water has been removed. Ca. 20 ml of toluene is distilled off. To the remaining solution of the imine of 1-[4-methoxy-3-(3-methoxypropyloxy)-phenyl]-3-methyl-butan-1-one are added at 0 °C first of all 23.5 ml of a 1-molar solution of bis-trimethylsilyl-lithium amide in tetrahydrofuran and then 5.7 g of dimethylpropylene urea. The solution obtained is added dropwise at 0°C to a solution of 8 g of trans-1-bromo-6-[4-(S)-benzyl-2-oxo-oxazolidin-3-yl]-5-(S)-isopropyl-hex-2-en-6-one in 10 ml of tolulene. After stirring, the mixture is acidified with diluted hydrochloric acid, separated, and the organic solution concentrated. The crude product is purified by chromatography on a column of silica gel (eluant: hexane/ethyl acetate 85:15).

### Example 8: trans-1-[4-(S)-benzyl-2-oxo-oxazolidin-3-yl]-8-[4-methoxy-3-(3-methoxypropyloxyl-phenyl]-2(S)-7(S)-diisopropyl-oct-4-en-1,8-dione

To a solution of 5.6 g of N-isovaleroyl-(S)-4-benzyl-oxazolidin-2-one in 12 ml of toluene are added, at 0°C, first of all 23.5 ml of a 1-molar solution of bis-trimethylsilyl-lithium amide in tetrahydrofuran, then 5.7 g of dimethylpropylene urea. The solution obtained is added dropwise at 0°C to a solution of 8 g of trans-1-bromo-6-[4-methoxy-3-(3-methoxypropyloxy)-phenyl]-5-(S)-isopropyl-hex-2-en-6-one in 10 ml of tolulene. After stirring, the mixture is acidified with diluted hydrochloric acid, separated, and the organic solution concentrated. The crude product is purified by chromatography on a column of silica gel (eluant: hexane/ethyl acetate 85:15).

### Example 9: 3(S)-isopropyl-5(S)-{1(R)-bromo-3(S)-isopropyl-4-[4-methoxy-3-(3-methoxypropoxy)phenyl]-4-oxo-butyl}-tetrahydrofuran-2-one

3.6 g of N-bromosuccinimide are added at room temperature to a solution of 11 g of trans-1-[4-(S)-benzyl-2-oxo-oxazolidin-3-yl]-8-[4-methoxy-3-(3-methoxypropyloxy)-phenyl]-2(S)-7(S)-diisopropyl-oct-4-en-1,8-dione in 50 ml of methylene chloride and 50 ml of water. The mixture is stirred for 2 hours, then the organic phase is separated and concentrated. The crude product is purified by chromatography on a column of silica gel (eluant: hexane/ethyl acetate 85:15).

### Example 10: 3(S)-Isopropyl-5(S)-{1(S)-azido-3(S)-isopropyl-4-[4-methoxy-3-(3-methoxypropyloxyl-phenyl]-4-oxo-butyl}-tetrahydrofuran-2-one

A well stirred mixture of 25 g of 3(S)-isopropyl-5(S)-{1(R)-bromo-3(S)-isopropyl-4-[4-methoxy-3-(3-methoxypropyloxy)-phenyl]-4-oxo-butyl}-tetrahydrofuran-2-one, 12.5g of sodium azide, 260 ml of toluene, 1.1 g of N-methyl-N,N,N-tricapryl-ammonium chloride and 38 ml of water is held at 75°C for 40 hours. Then, the mixture is cooled to 20°C, the organic solution separated, washed with an aqueous solution of sodium nitrate and acetic acid, and concentrated.

### Example 11: 3(S)-Isopropyl-5(S)-{1(S)-amino-3(S)-isopropyl-4-[4-methoxy-3-(3-methoxypropyloxy)-phenyl]-butyl}tetrahydrofuran-2-one

A solution of 11 g of 3(S)-isopropyl-5(S)-{1(S)-azido-3(S)-isopropyl-4-[4-methoxy-3-(3-methoxypropyloxy)-phenyl]-4-oxo-butyl}-tetrahydrofuran-2-one and 1.2 ml ethanolamin in 670 ml of ethanol is hydrogenated for 24 hours at room temperature and at normal pressure in the presence of 11 g of palladium on activated carbon (10%). After filtering the catalyst, the mixture is concentrated and the residue partitioned between aqueous sodium hydrogen carbonate solution and toluene. After concentrating the toluene, the product is obtained as a colourless resin.

## Claims

1. Compounds of formula I 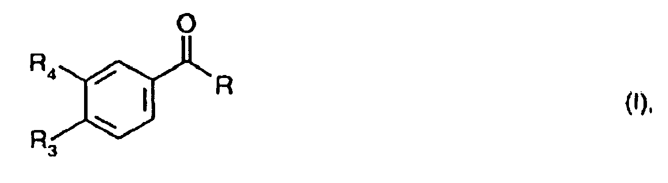 wherein
R is a group of formulae Ia, Ib, Ic or Id 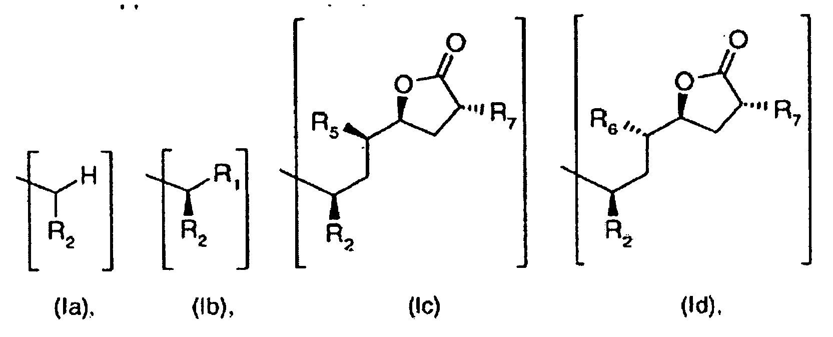 R₁ is 4-halogenbut-2-enyl,
R₂ is C₁-C₇- alkyl or cycloalkyl,
R₃ is C₁-C₇- alkoxy,
R₄ is C₁-C₇- alkoxy C₁-C₇- alkoxy, or where R is a group of formula (Ia), it is hydroxy, hydroxy C₁-C₇- alkoxy or a group of formula Ie 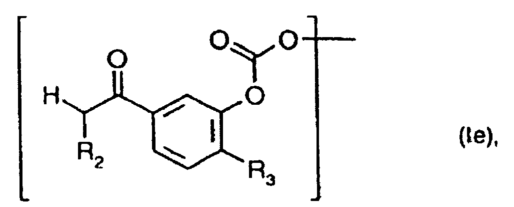 R₅ is reactive, esterified hydroxyl ,
R₆ is azido and
R₇ is C₁-C₇- alkyl, C₁-C₇- alkenyl, cycloalkyl or aryl C₁-C₇- alkyl,
and their salts, wherein reactive esterified hydroxyl is selected from the group consisting of halogen or C₁-C₇- alkanesulfonyloxy, halogen C₁-C₇- alkanesulfonyl,; or benzenesulfonyloxy or naphthalenesulfonyloxy either unsubstituted or substituted by C₁-C₇-alkyl, halogen and/or nitro.

2. Compounds according to claim 1, wherein
R is a group of formulae la, Ib, Ic or Id,
R₁ is 4-halogenbut-2-enyl ,
R₂ is C₁-C₇- alkyl or 3- to 8-membered cycloalkyl,
R₃ is C₁-C₇- alkoxy,
R₄ is C₁-C₇- alkoxy C₁-C₇- alkoxy, or where R is a group of formula (Ia), it is hydroxy, hydroxy C₁-C₇- alkoxy or a group of formula Ie,
R₅ is halogen or C₁-C₇- alkane sulfonyloxy, halogen C₁-C₇- alkane sulfonyl; or benzene sulfonyloxy or naphthalene sulfonyloxy which is unsubstituted or substituted by C₁-C₇- alkyl, halogen and/or nitro.
R₆ is azido and
R₇ is C₁-C₇- alkyl, C₁-C₇- alkenyl, 3- to 8-membered cycloalkyl, or phenyl C₁-C₇- alkyl which is unsubstituted or substituted in the phenyl moiety by C₁-C₇- alkyl, C₁-C₇- alkoxy, halogen and/or nitro,
and their salts.

3. Compounds according to claim 1, wherein
R is a group of formulae Ia, Ib, Ic or Id,
R₁ is 4-halogenbut-2-enyl ,
R₂ is branched C₃-C₇-alkyl, such as propyl,
R₃ is C₁-C₄-alkoxy, such as methoxy,
R₄ is C₁-C₄-alkoxy-C₂-C₄-alkoxy, such as 3-methoxypropyloxy, or where R is a group of formula (Ia), it is hydroxy, hydroxy-C₂-C₄-alkoxy, such as 3-hydroxypropyloxy, or a group of formula Ie,
R₅ is halogen of atomic numbers up to and including 35, such as bromine,
R₆ is azido or amino and
R₇ is branched C₃-C₇-alkyl, such as propyl,
and their salts.

4. A compound according to claim 1, selected from
Bis-(3-isovaleroyl-6-methoxy-phenyl)-carbonate;
1-(3-hydroxy-4-methoxy-phenyl)-3-methyl-butan-1-one;
1-[3-(3-hydroxypropyloxy)-4-methoxy-phenyl]-3-methyl-butan-1-one
1-[4-methoxy 3-(3-methoxypropyloxy)-phenyl]-3-methyl-butan-1-one;
*trans*-1-bromo-6-[4-methoxy-3-(3-methoxypropyloxy)-phenyl]-5-(S)-isopropyl-hex-2-en-6-one;
3(S)-isopropyl-5(S)-{1(R)-bromo-3(S)-isopropyl-4-[4-methoxy-3-(3-methoxypropoxy)phenyl]-4-oxo-butyl}-tetrahydrofuran-2-one and
3(S)-isopropyl-5(S)-{1(S)-azido-3(S)-isopropyl-4-[4-methoxy-3-(3-methoxypropyloxy)phenyl]-4-oxo-butyl}-tetrahydrofuran-2-one.

5. Method of producing compounds of formula I 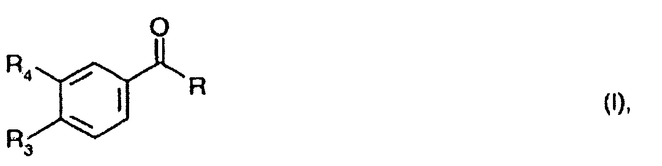 wherein
R is a group of formulae la, Ib, Ic or Id 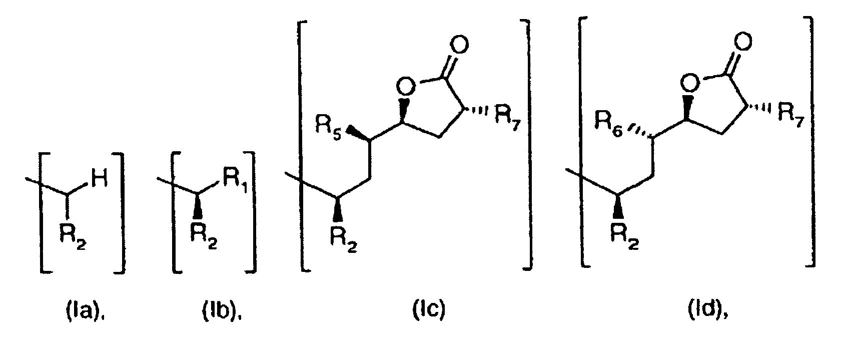 R₁ is 4-halogenbut-2-enyl ,
R₂ is C₁-C₇- alkyl or cycloalkyl,
R₃ is C₁-C₇- alkoxy,
R₄ is C₁-C₇- alkoxy C₁-C₇- alkoxy, or where R is a group of formula (Ia), it is hydroxy, hydroxy C₁-C₇- alkoxy or a group of formula Ie 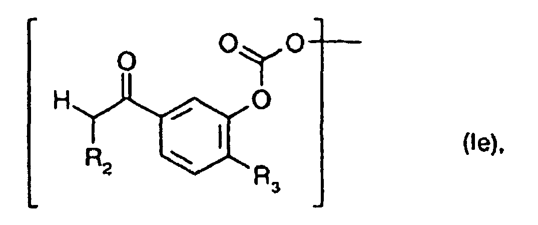 R₅ is reactive, esterified hydroxyl as defined in claim 1,
R₆ is azido and
R₇ is C₁-C₇- alkyl, C₁-C₇- alkenyl, cycloalkyl or aryl C₁-C₇- alkyl,
and their salts, **characterised in that**
a) in order to produce compounds of formula I, wherein R is a group of formula Ia, R₂ is C₁-C₇- alkyl or cycloalkyl, R₃ is C₁-C₇- alkoxy and R₄ is a group of formula Ie,
a compound of formula IV 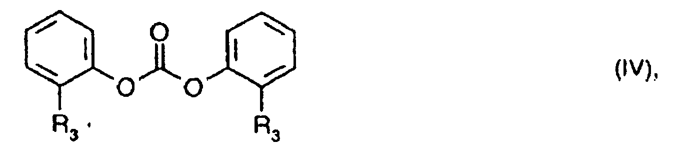 wherein R₃ has the above significance, is condensed with a compound of formula V 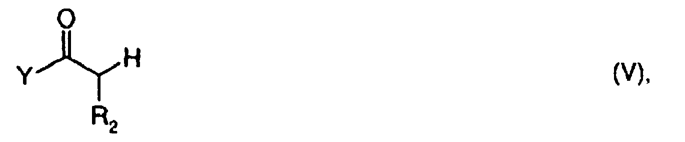 wherein R₂ has the above significance and Y is a reactive esterified hydroxy group,
b) in order to produce a compound of formula I, wherein R is a group of formula la, R₂ is C₁-C₇- alkyl or cycloalkyl, R₃ is C₁-C₇- alkoxy and R₄ is hydroxy, a compound of formula VI, 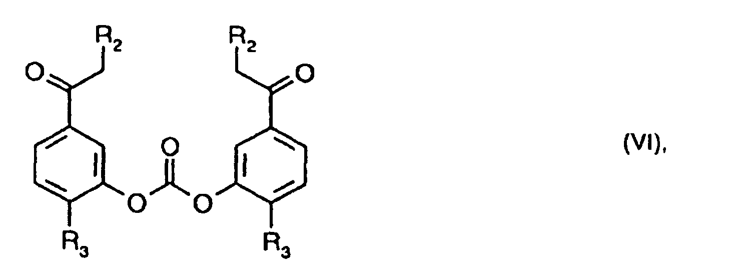 wherein R₂ and R₃ have the above significances, undergoes solvolysis to form the corresponding compounds of formula I, wherein R₄ is hydroxy,
c) in order to produce a compound of formula I, wherein R is a group of formula Ia, R₂ is C₁-C₇- alkyl or cycloalkyl, R₃ is C₁-C₇- alkoxy and R₄ is hydroxy C₁-C₇- alkoxy R'₄, in a compound
of formula VII, 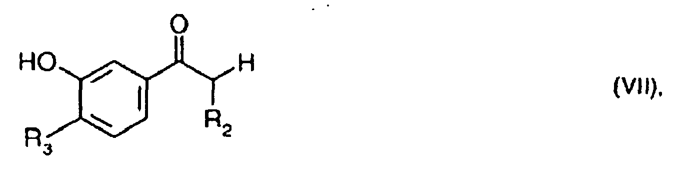 wherein R₂ and R₃ have the above significances, the hydroxy group is converted into hydroxy C₁-C₇- alkoxy R'₄,
d) in order to produce a compound of formula I, wherein R is a group of formula la, R₂ is C₁-C₇- alkyl or cycloalkyl, R₃ is C₁-C₇- alkoxy and R₄ is C₁-C₇- alkoxy C₁-C₇- alkoxy R"₄, in a
compound of formula VIII, 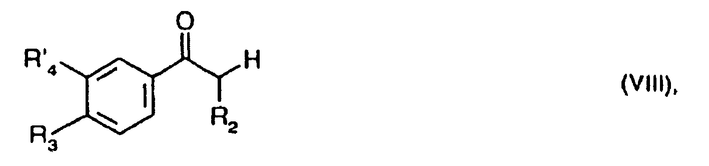 wherein R₂ and R₃ have the above significances and R'₄ is hydroxy C₁-C₇- alkoxy, the hydroxy C₁-C₇- alkoxy group is converted into C₁-C₇- alkoxy C₁-C₇- alkoxy R"₄,
e) in order to produce compounds of formula I, wherein R is a group of formula Ib, R₁ is 4-halogen-but-2-enyl, R₂ is C₁-C₇- alkyl or cycloalkyl, R₃ is C₁-C₇- alkoxy and R₄ is C₁-C₇-alkoxy C₁-C₇- alkoxy R"₄, a compound of formula IX 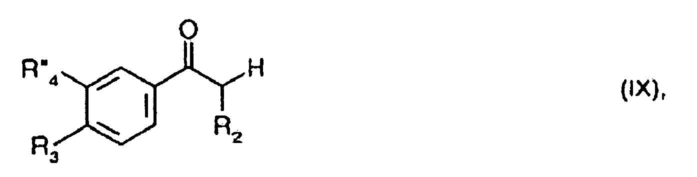 wherein R₂, R₃ and R"₄ have the above significances, is reacted first of all with a chiral primary amine which is customary for the purpose of stereoselective synthesis, and then with a 1,4-dihalogen-but-2-ene,
f) in order to produce compounds of formula I, wherein R is a group of formula Ic, R₂ is C₁-C₇- alkyl or cycloalkyl, R₃ is C₁-C₇- alkoxy, R₄ is C₁-C₇- alkoxy C₁-C₇- alkoxy R"₄, R₅ is reactive esterified hydroxy and R₇ is C₁-C₇- alkyl, C₁-C₇- alkenyl, cycloalkyl or aryl C₁-C₇-alkyl,
a compound of formula X, 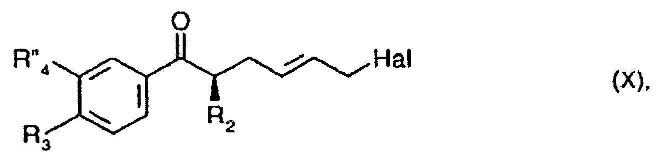 wherein R₂, R₃ and R"₄ have the above significances, and Hal is halogen, is reacted with a compound of formula XI 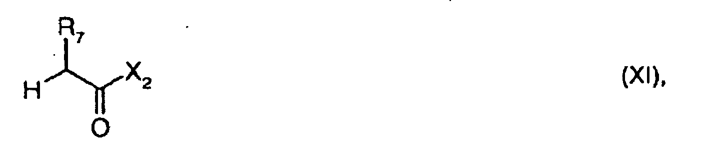 in which R₇ has the indicated significance and X₂ is a chiral amino, amido or urethane group which is customary for the purpose of stereoselective synthesis, or is a chiral alcohol group; or a 1,4-dihalogen-but-2-ene is reacted firstly with a compound of formula XI and then with the reaction product from the reaction of a compound of formula IX 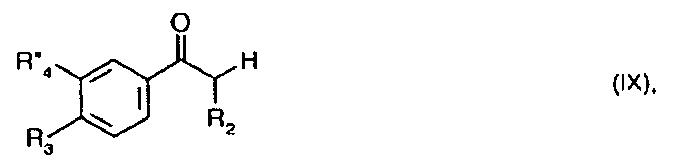 with a chiral primary amine of formula XII, which is customary for the purpose of stereoselective synthesis, 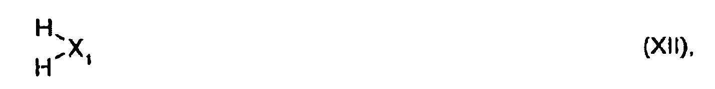 and the reaction product of formula XIII 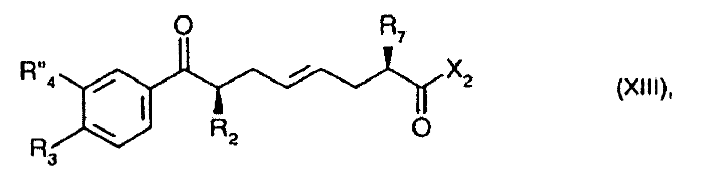 is condensed intramolecularly with lactonisation to form the corresponding compounds of formula I, wherein R is a group of formula Ic and R₅ is halogen, and if desired, halogen R₅ is converted stereoselectively into another reactive etherified hydroxy group R₅, and/or
g) in order to produce compounds of formula I, wherein R is a group of formula Id, R₂ is C₁-C₇- alkyl or cycloalkyl, R₃ is C₁-C₇- alkoxy, R₄ is C₁-C₇- alkoxy C₁-C₇- alkoxy R"₄, R₆ is azido and R₇ is C₁-C₇- alkyl, C₁-C₇- alkenyl, cycloalkyl or aryl C₁-C₇- alkyl, in a compound of formula XIV 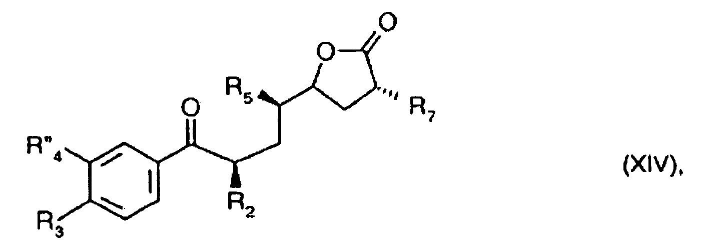 wherein R₂, R₃, R"₄ and R₇ have the above significances, and R₅ is reactive esterified hydroxyl as defined in claim 1, reactive esterified hydroxy R₅ is replaced stereospecifically by azido, and if desired, a free compound obtainable according to the method is converted into a salt, or a salt obtainable according to the method is converted into the free compound or into another salt.

6. Method of producing compounds of formula II 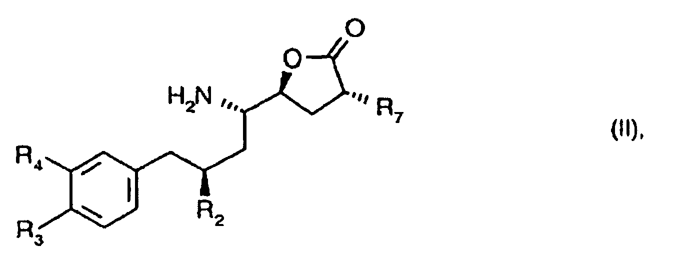 wherein
R₂ is C₁-C₇- alkyl or cycloalkyl,
R₃ is C₁-C₇- alkoxy,
R₄ is C₁-C₇- alkoxy C₁-C₇- alkoxy and
R₇ is C₁-C₇- alkyl, C₁-C₇- alkenyl, cycloalkyl or aryl C₁-C₇- alkyl,
**characterised in that**
a) a compound of formula IV, 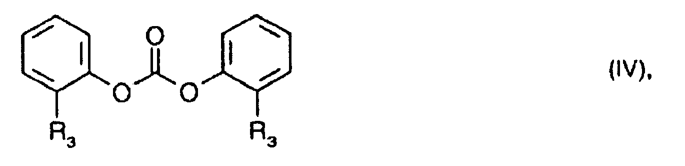 wherein R₃ has the above significance, is condensed with a compound of formula V 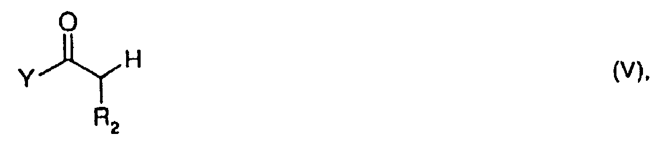 wherein R₂ has the above significance and Y is a reactive esterified hydroxy group,
b) in the obtained compound of formula VI 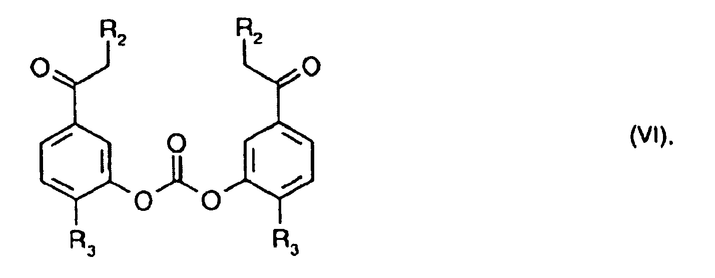 wherein R₂ and R₃ have the above significances, undergoes solvolysis to form the corresponding compounds of formula I, wherein R₄ is hydroxy,
c) in the obtained compounds of formula VII 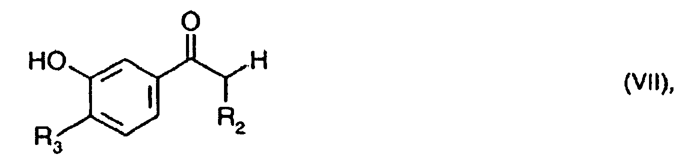 wherein R₂ and R₃ have the above significances, the hydroxy group is converted into hydroxy C₁-C₇- alkoxy R'₄,
d) in the obtained compounds of formula VIII 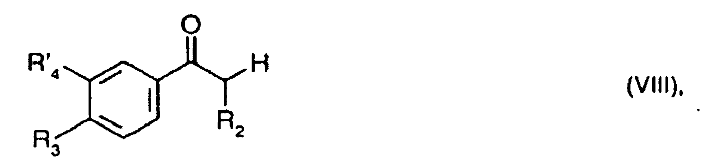 wherein R₂ and R₃ have the above significances and R'₄ is hydroxy C₁-C₇- alkoxy, the hydroxy C₁-C₇- alkoxy group is converted into C₁-C₇- alkoxy C₁-C₇- alkoxy R"₄,
e) the obtained compound of formula IX 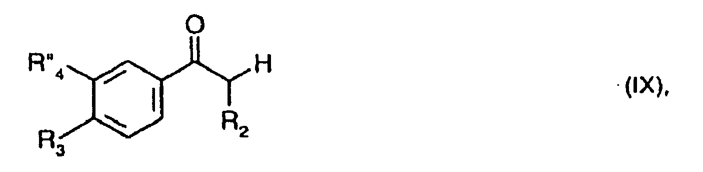 wherein R₂, R₃ and R"₄ have the above significances, is reacted first of all with a chiral primary amine which is customary for the purpose of stereoselective synthesis, and then with a 1,4-dihalogen-but-2-ene,
f) the obtained compound of formula X 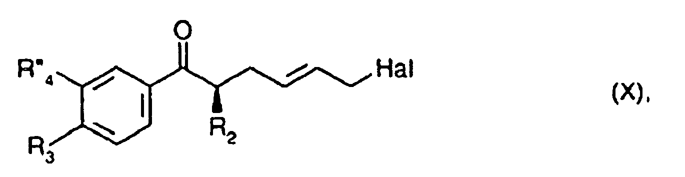 wherein R₂, R₃ and R"₄ have the above significances, and Hal is halogen, is reacted with a compound of formula XI 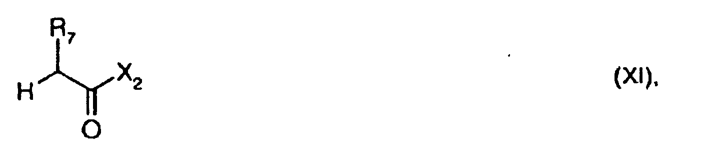 in which R₇ has the indicated significance and X₂ is a chiral amino, amido or urethane group which is customary for the purpose of stereoselective synthesis, or is a chiral alcohol group; or a 1,4-dihalogen-but-2-ene is reacted firstly with a compound of formula XI and then with the reaction product from the reaction of a compound of formula IX 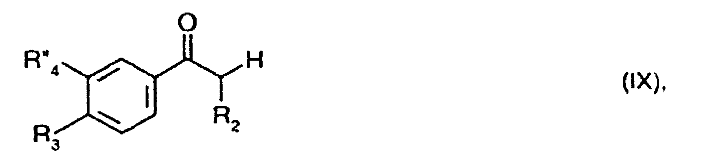 with a chiral primary amine of formula XII, which is customary for the purpose of stereoselective synthesis, 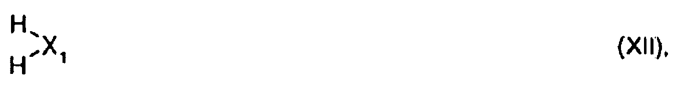 and the reaction product of formula XIII 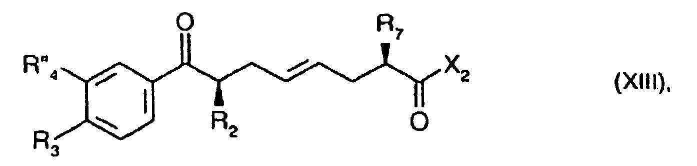 is condensed intramolecularly with lactonisation to form the corresponding compounds of formula I, wherein R is a group of formula Ic and R₅ is halogen, and if desired, halogen is converted stereoselectively into another reactive etherified hydroxy group R₅ as defined in claim 1,
g) in the obtained compounds of formula XIV 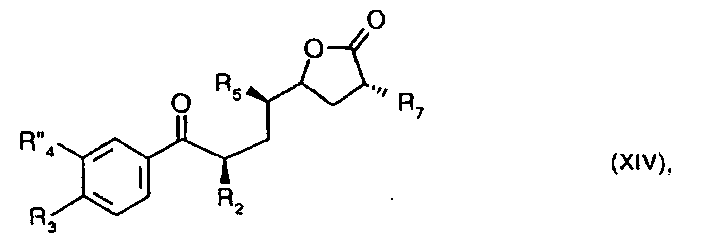 wherein R₂, R₃, R"₄ and R₇ have the above significances, and R₅ is reactive esterified hydroxyl as defined in claim 1, reactive esterified hydroxy R₅ is replaced stereospecifically by azido, and
h) in a compound of formula I, wherein R is a group of formula Id, R₂ is C₁-C₇- alkyl or cycloalkyl, R₃ is C₁-C₇- alkoxy, R₄ is C₁-C₇- alkoxy C₁-C₇- alkoxy, R₆ is azido and R₇ is C₁-C₇-alkyl, C₁-C₇- alkenyl, cycloalkyl or aryl C₁-C₇- alkyl, the azido group R₆ is reduced to amino, and at the same time or subsequently, the benzoyl group is reduced to the corresponding benzyl group, and if desired, a free compound obtainable according to the method is converted into a salt, or a salt obtainable according to the method is converted into the free compound or into another salt.

## Patentansprüche

1. Verbindungen der Formel I 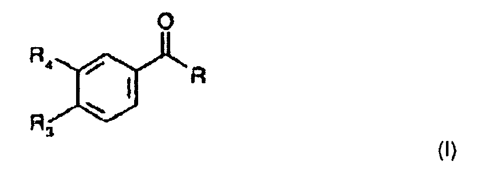 worin R für eine Gruppe der Formeln la, Ib, Ic oder Id steht 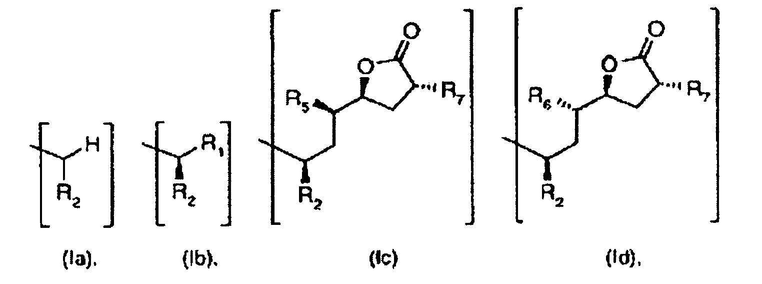 R₁ für 4-Halogenbut-2-enyl steht,
R₂ für C₁-C₇-Alkyl oder Cycloalkyl steht,
R₃ für C₁-C₇-Alkoxy steht,
R₄ für C₁-C₇-Alkoxy-C₁-C₇-alkoxy steht oder, wenn R für eine Gruppe der Formel (Ia) steht, dann für Hydroxy, Hydroxy-C₁-C₇-alkoxy oder eine Gruppe der Formel Ie steht 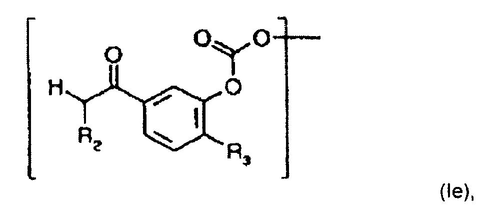 R₅ für reaktives, verestertes Hydroxyl steht,
R₆ für Azido steht und
R₇ für C₁-C₇-Alkyl, C₁-C₇-Alkenyl, Cycloalkyl oder Aryl-C₁-C₇-alkyl steht,
und die Salze hiervon, worin reaktives verestertes Hydroxyl aus der Gruppe ausgewählt ist, die aus Halogen oder C₁-C₇-Alkansulfonyloxy, Halogen-C₁-C₇-alkansulfonyl; oder Benzolsulfonyloxy oder Naphthalinsulfonyloxy, das entweder nicht substituiert oder mit C₁-C₇-Alkyl, Halogen und/oder Nitro substituiert ist, besteht.

2. Verbindungen nach Anspruch 1, worin
R für eine Gruppe der Formeln Ia, Ib, Ic oder Id steht,
R₁ für 4-Halogenbut-2-enyl steht,
R₂ für C₁-C₇-Alkyl oder ein 3- bis 8-gliedriges Cycloalkyl steht,
R₃ für C₁-C₇-Alkoxy steht,
R₄ für C₁-C₇-Alkoxy-C₁-C₇-alkoxy steht oder, wenn R für eine Gruppe der Formel (Ia) steht, dann für Hydroxy, Hydroxy-C₁-C₇-alkoxy oder eine Gruppe der Formel Ie steht,
R₅ für Halogen oder C₁-C₇-Alkansulfonyloxy, Halogen-C₁-C₇-alkansulfonyl; oder Benzolsulfonyloxy oder Naphthalinsulfonyloxy, das nicht substituiert oder durch C₁-C₇-Alkyl, Halogen und/oder Nitro substituiert ist, steht,
R₆ für Azido steht und
R₇ für C₁-C₇-Alkyl, C₁-C₇-Alkenyl, 3- bis 8-gliedriges Cycloalkyl oder Phenyl-C₁-C₇-alkyl steht, das nicht substituiert oder in der Phenyleinheit durch C₁-C₇-Alkyl, C₁-C₇-Alkoxy, Halogen und/oder Nitro substituiert ist,
und die Salze hiervon.

3. Verbindungen nach Anspruch 1, worin
R für eine Gruppe der Formeln Ia, Ib, Ic oder Id steht,
R₁ für 4-Halogenbut-2-enyl steht,
R₂ für verzweigtes C₃-C₇-Alkyl, wie Propyl, steht,
R₃ für C₁-C₄-Alkoxy, wie Methoxy, steht,
R₄ für C₁-C₄-Alkoxy-C₂-C₄-alkoxy, wie 3-Methoxypropyloxy steht oder, wenn R für eine Gruppe der Formel (Ia) steht, dann für Hydroxy, Hydroxy-C₂-C₄-alkoxy, wie 3-Hydroxypropyloxy oder eine Gruppe der Formel Ie steht,
R₅ für Halogen, dessen Atomzahl bis zu und einschließlich 35 beträgt, wie Brom, steht,
R₆ für Azido oder Amino steht und
R₇ für verzweigtes C₃-C₇-Alkyl, wie Propyl, steht
und die Salze hiervon.

4. Verbindung nach Anspruch 1, die aus den folgenden Verbindungen ausgewählt ist:
Bis-(3-isovaleroyl-6-methoxy-phenyl)-carbonat;
1-(3-Hydroxy-4-methoxy-phenyl)-3-methyl-butan-1-on;
1-[3-(3-Hydroxypropyloxy)-4-methoxy-phenyl]-3-methyl-butan-1-on;
1-[4-Methoxy-3-(3-methoxypropyloxy)-phenyl]-3-methyl-butan-1-on;
trans-1-Brom-6-[4-methoxy-3-(3-methoxypropyloxy)-phenyl]-5-(S)-isopropyl-hex-2-en-6-on;
3(S)-Isopropyl-5(S)-{1(R)-brom-3(S)-isopropyl-4-[4-methoxy-3-(3-methoxypropoxy)phenyl]-4-oxo-butyl}-tetrahydrofuran-2-on und
3(S)-Isopropyl-5(S)-{1(S)-azido-3(S)-isopropyl-4-[4-methoxy-(3-methoxypropyloxy)phenyl]-4-oxo-butyl}-tetrahydrofuran-2-on.

5. Verfahren zur Herstellung von Verbindungen der Formel I 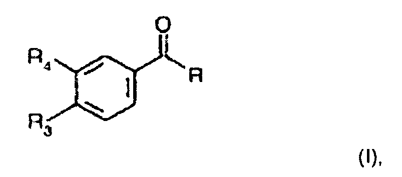 worin R für eine Gruppe der Formeln Ia, Ib, Ic oder Id steht 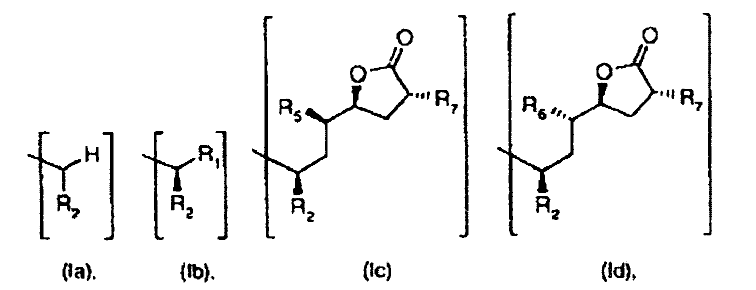 R₁ für 4-Halogenbut-2-enyl steht,
R₂ für C₁-C₇-Alkyl oder Cycloalkyl steht,
R₃ für C₁-C₇-Alkoxy steht,
R₄ für C₁-C₇-Alkoxy-C₁-C₇-alkoxy steht oder, wenn R für eine Gruppe der Formel (Ia) steht, dann für Hydroxy, Hydroxy-C₁-C₇-alkoxy oder eine Gruppe der Formel Ie steht, 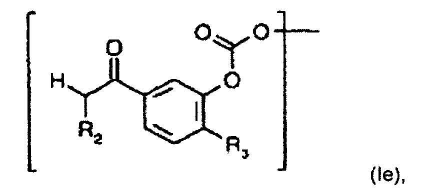 R₅ für reaktives verestertes Hydroxyl gemäß Definition in Anspruch 1 steht,
R₆ für Azido steht und
R₇ für C₁-C₇-Alkyl, C₂-C₇-Alkenyl, Cycloalkyl oder Aryl-C₁-C₇-alkyl steht,
und den Salzen hiervon, **dadurch gekennzeichnet, dass**
a) zur Herstellung von Verbindungen der Formel I, worin R für eine Gruppe der Formel Ia steht, R₂ für C₁-C₇-Alkyl oder Cycloalkyl steht, R₃ für C₁-C₇-Alkoxy steht und R₄ für eine Gruppe der Formel Ie steht, eine Verbindung der Formel IV 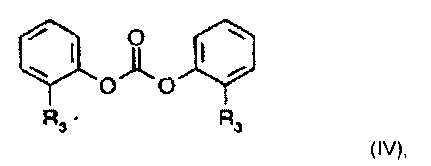 worin R₃ die oben angegebene Bedeutung besitzt, mit einer Verbindung der Formel V kondensiert wird, 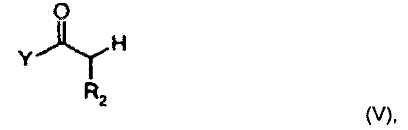 worin R₂ die oben angegebene Bedeutung besitzt und Y für eine reaktive veresterte Hydroxygruppe steht,
b) zur Herstellung einer Verbindung der Formel I, worin R für eine Gruppe der Formel Ia steht, R₂ für C₁-C₇-Alkyl oder Cycloalkyl steht, R₃ für C₁-C₇-Alkoxy steht und R₄ für Hydroxy steht, eine Verbindung der Formel VI 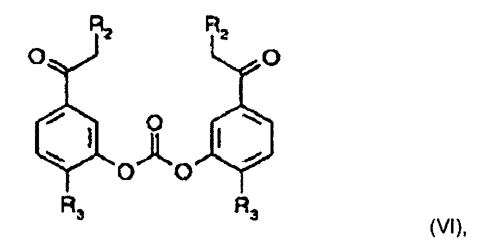 worin R₂ und R₃ die oben angegebenen Bedeutungen besitzen, einer Solvolyse unterzogen wird, um die entsprechenden Verbindungen der Formel I zu bilden, worin R₄ für Hydroxy steht,
c) zur Herstellung einer Verbindung der Formel I, worin R für eine Gruppe der Formel Ia steht, R₂ für C₁-C₇-Alkyl oder Cycloalkyl steht, R₃ für C₁-C₇-Alkoxy steht und R₄ für Hydroxy-C₁-C₇-alkoxy (R'₄) steht, in einer Verbindung der Formel VII 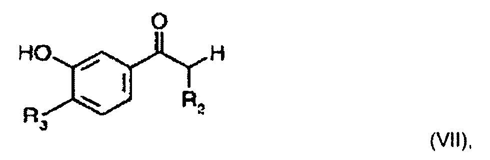 worin R₂ und R₃ die oben angegebenen Bedeutungen besitzen, die Hydroxygruppe in Hydroxy-C₁-C₇-alkoxy (R'₄) umgewandelt wird,
d) zur Herstellung einer Verbindung der Formel I, worin R für eine Gruppe der Formel Ia steht, R₂ für C₁-C₇-Alkyl oder Cycloalkyl steht, R₃ für C₁-C₇-Alkoxy steht und R₄ für C₁-C₇-Alkoxy-C₁-C₇-alkoxy (R"₄) steht, in einer Verbindung der Formel VIII 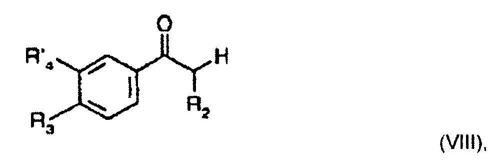 worin R₂ und R₃ die oben angegebenen Bedeutungen besitzen und R'₄ für Hydroxy-C₁-C₇-alkoxy steht, die Hydroxy-C₁-C₇-alkoxygruppe in C₁-C₇-Alkoxy-C₁-C₇-alkoxy (R"₄) umgewandelt wird,
e) zur Herstellung von Verbindungen der Formel I, worin R für eine Gruppe der Formel Ib steht, R₁ für 4-Halogenbut-2-enyl steht, R₂ für C₁-C₇-Alkyl oder Cycloalkyl steht, R₃ für C₁-C₇-Alkoxy steht und R₄ für C₁-C₇-Alkoxy-C₁-C₇-alkoxy (R"₄) steht, eine Verbindung der Formel IX 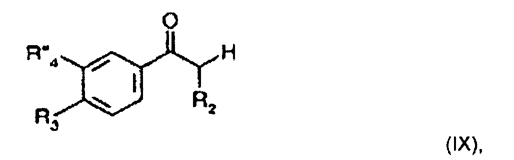 worin R₂, R₃ und R"₄ die oben angegebenen Bedeutungen besitzen, zuerst mit einem chiralen primären Amin, das für die Zwecke einer stereoselektiven Synthese üblich ist, und anschließend mit einem 1,4-Dihalogenbut-2-en umgesetzt wird,
f) zur Herstellung von Verbindungen der Formel I, worin R für eine Gruppe der Formel Ic steht, R₂ für C₁-C₇-Alkyl oder Cycloalkyl steht, R₃ für C₁-C₇-Alkoxy steht, R₄ für C₁-C₇-Alkoxy-C₁-C₇-alkoxy (R"₄) steht, R₅ für reaktives verestertes Hydroxy steht und R₇ für C₁-C₇-Alkyl, C₁-C₇-Alkenyl, Cycloalkyl oder Aryl-C₁-C₇-alkyl steht, eine Verbindung der Formel X 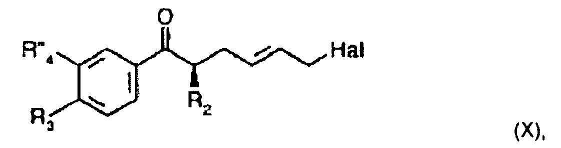 worin R₂, R₃ und R"₄ die oben angegebenen Bedeutungen besitzen und HaI für Halogen steht, mit einer Verbindung der Formel XI umgesetzt wird 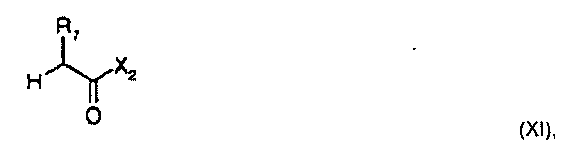 worin R₇ die oben angegebene Bedeutung besitzt und X₂ für eine chirale Amino-, Amido- oder Urethangruppe, die für die Zwecke einer stereoselektiven Synthese üblich ist, oder eine chirale Alkoholgruppe steht; oder 1,4-Dihalogenbut-2-en zuerst mit einer Verbindung der Formel XI und anschließend mit dem Reaktionsprodukt aus der Reaktion einer Verbindung der Formel IX 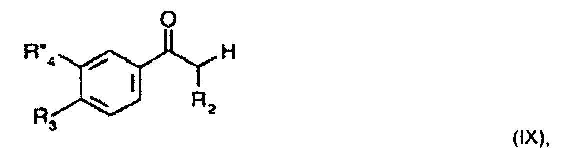 mit einem chiralen primären Amin der Formel XII, das für die Zwecke einer stereoselektiven Synthese üblich ist, 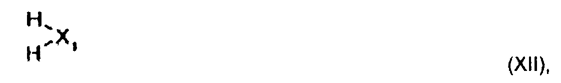 und das Reaktionsprodukt der Formel XIII 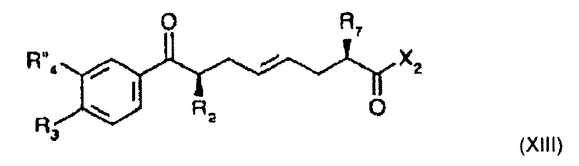 intramolekular unter Lactonisierung kondensiert wird, wobei die entsprechenden Verbindungen der Formel I gebildet werden, worin R für eine Gruppe der Formel Ic steht und R₅ für Halogen steht, und, falls gewünscht, Halogen (R₅) stereoselektiv in eine andere reaktive veretherte Hydroxygruppe R₅ umgewandelt wird und/oder
g) zur Herstellung von Verbindungen der Formel I, worin R für eine Gruppe der Formel Id steht, R₂ für C₁-C₇-Alkyl oder Cycloalkyl steht, R₃ für C₁-C₇-Alkoxy steht, R₄ für C₁-C₇-Alkoxy-C₁-C₇-alkoxy (R"₄) steht, R₆ für Azido steht und R₇ für C₁-C₇-Alkyl, C₁-C₇-Alkenyl, Cycloalkyl oder Aryl-C₁-C₇-alkyl steht, in einer Verbindung der Formel XIV 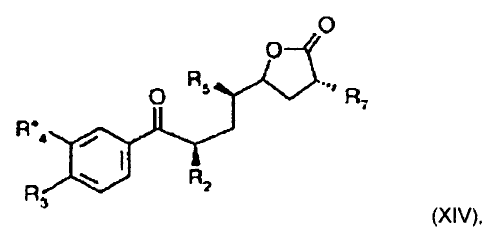 worin R₂, R₃, R"₄ und R₇ die oben angegebenen Bedeutungen besitzen und R₅ für ein reaktives verestertes Hydroxyl gemäß Definition in Anspruch 1 steht, reaktives verestertes Hydroxy (R₅) stereospezifisch durch Azido ersetzt wird und, falls gewünscht, eine nach dem Verfahren erhältliche freie Verbindung in ein Salz oder ein nach dem Verfahren erhältliches Salz in die freie Verbindung oder ein anderes Salz umgewandelt wird.

6. Verfahren zur Herstellung von Verbindungen der Formel II 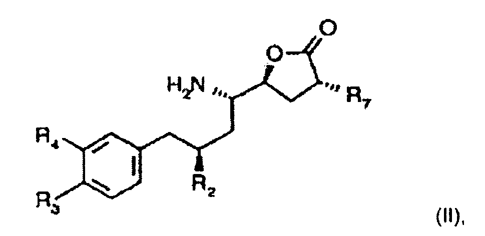 worin R₂ für C₁-C₇-Alkyl oder Cycloalkyl steht,
R₃ für C₁-C₇-Alkoxy steht,
R₄ für C₁-C₇-Alkoxy-C₁-C₇-alkoxy steht und
R₇ für C₁-C₇-Alkyl, C₁-C₇-Alkenyl, Cycloalkyl oder Aryl- C₁-C₇-alkyl steht, das **dadurch gekennzeichnet ist, dass**
a) eine Verbindung der Formel IV 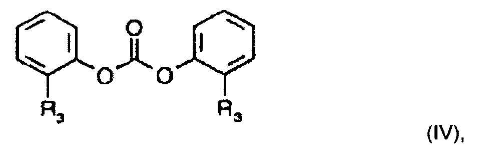 worin R₃ die oben angegebene Bedeutung besitzt, mit einer Verbindung der Formel V 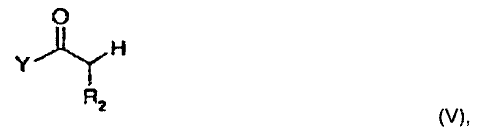 worin R₂ die oben angegebene Bedeutung besitzt und Y für eine reaktive veresterte Hydroxygruppe steht, kondensiert wird,
b) die erhaltene Verbindung der Formel VI 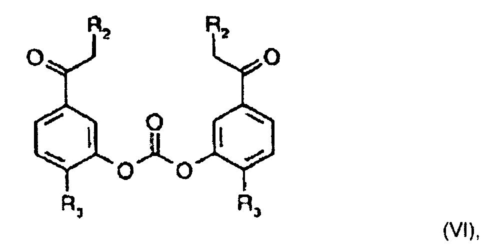 worin R₂ und R₃ die oben angegebenen Bedeutungen besitzen, einer Solvolyse unterzogen wird, wobei die entsprechenden Verbindungen der Formel I gebildet werden, worin R₄ für Hydroxy steht,
c) in den erhaltenen Verbindungen der Formel VII 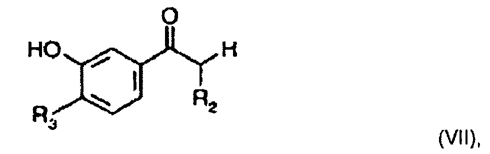 worin R₂ und R₃ die oben angegebenen Bedeutungen besitzen, die Hydroxygruppe in Hydroxy-C₁-C₇-alkoxy (R'₄) umgewandelt wird,
d) in den erhaltenen Verbindungen der Formel VIII 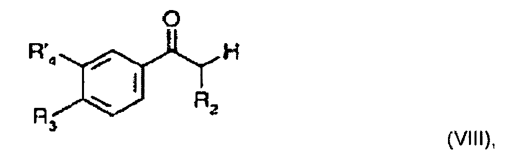 worin R₂ und R₃ die oben angegebenen Bedeutungen besitzen und R'₄ für Hydroxy-C₁-C₇-alkoxy steht, die Hydroxy-C₁-C₇-alkoxygruppe in C₁-C₇-Alkoxy-C₁-C₇-alkoxy (R"₄) umgewandelt wird,
e) die erhaltene Verbindung der Formel IX 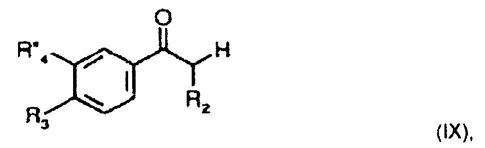 worin R₂, R₃ und R"₄ die oben angegebenen Bedeutungen besitzen, zuerst mit einem chiralen primären Amin, das für die Zwecke einer stereoselektiven Synthese üblich ist, und anschließend mit einem 1,4-Dihalogenbut-2-en umgesetzt wird,
f) die erhaltene Verbindung der Formel X 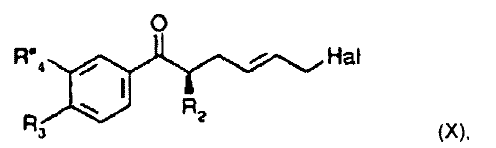 worin R₂, R₃ und R"₄ die oben angegebenen Bedeutungen besitzen und HaI für Halogen steht, mit einer Verbindung der Formel XI umgesetzt wird 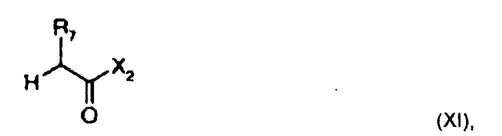 worin R₇ die oben angegebene Bedeutung besitzt und X₂ für eine chirale Amino-, Amido- oder Urethangruppe, die für die Zwecke einer stereoselektiven Synthese üblich ist, oder eine chirale Alkoholgruppe steht; oder 1,4-Dihalogenbut-2-en zuerst mit einer Verbindung der Formel XI und anschließend mit dem Reaktionsprodukt aus der Reaktion einer Verbindung der Formel IX 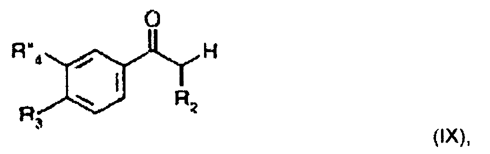 mit einem chiralen primären Amin der Formel XII, das für die Zwecke einer stereoselektiven Synthese üblich ist, umgesetzt wird 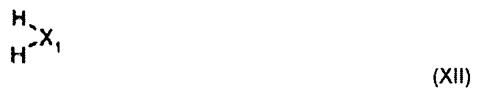 und das Reaktionsprodukt der Formel XIII 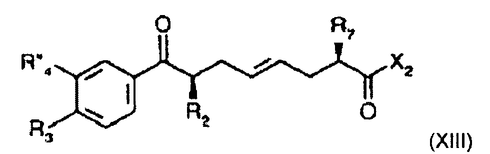 intramolekular unter Lactonisierung kondensiert wird, wobei die entsprechenden Verbindungen der Formel I gebildet werden, worin R für eine Gruppe der Formel Ic steht und R₅ für Halogen steht, und, falls gewünscht. Halogen stereoselektiv in eine andere reaktive veretherte Hydroxygruppe R₅ gemäß Definition in Anspruch 1 umgewandelt wird,
g) in den erhaltenen Verbindungen der Formel XIV 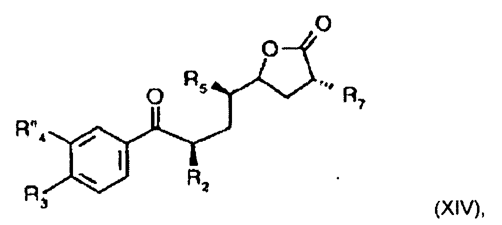 worin R₂, R₃, R"₄ und R₇ die oben angegebenen Bedeutungen besitzen und R₅ für ein reaktives verestertes Hydroxyl gemäß Definition in Anspruch 1 steht, reaktives verestertes Hydroxy (R₅) stereospezifisch durch Azido ersetzt wird und,
h) in einer Verbindung der Formel I, worin R für eine Gruppe der Formel Id steht, R₂ für C₁-C₇-Alkyl oder Cycloalkyl steht, R₃ für C₁-C₇-Alkoxy steht, R₄ für C₁-C₇-Alkoxy-C₁-C₇-alkoxy steht, R₆ für Azido steht und R₇ für C₁-C₇-Alkyl, C₁-C₇-Alkenyl, Cycloalkyl oder Aryl-C₁-C₇-alkyl steht, die Azidogruppe (R₆) zu Amino reduziert wird und gleichzeitig oder nachfolgend die Benzoylgruppe zu der entsprechenden Benzylgruppe reduziert wird und, falls gewünscht, eine nach dem Verfahren erhältliche freie Verbindung in ein Salz umgewandelt wird oder ein nach dem Verfahren erhältliches Salz in die freie Verbindung oder in ein anderes Salz umgewandelt wird.

## Revendications

1. Composés de formule I 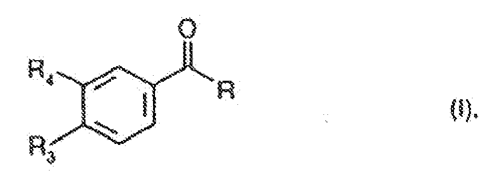 dans laquelle
R. est un groupe de formule Ia, Ib, Ie ou Id 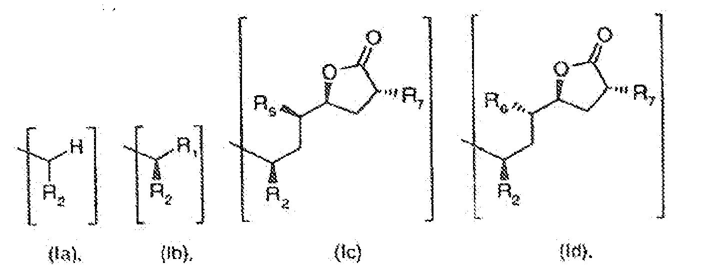 R₁ est un groupe 4-halogénobut-2-ényle,
R₂ est un groupe alkyle en C₁-C₇ ou cycloalkyle,
R₃ est un groupe alcoxy en C₁-C₇,
R₄ est un groupe alcoxy(en C₁-C₇)-alcoxy en C₁-C₇, ou si R est un groupe de formule (Ia), il s'agit d'un groupe hydroxy, hydroxy-alcoxy en C₁-C₇ ou d'un groupe de formule Ie 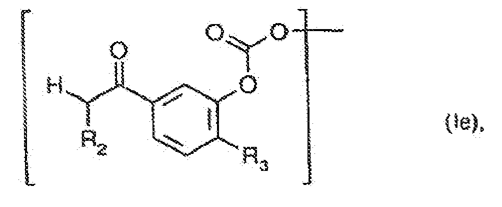 R₅ est un groupe hydroxyle estérifié réactif,
R₆ est un groupe azido, et
R₇ est un groupe alkyle en C₁-C₇, alcényle en C₁-C₇, cycloalkyle ou aryl-alkyle en C₁-C₇,
et leurs sels, où le groupe hydroxyle estérifié réactif est sélectionne dans le groupe composé d'un halogène ou d'un groupe (alcane en C₁-C₇)sulfonyloxy, halogène-(alcane en C₁-C₇)sulfonyle ; ou benzènesulfonyloxy ou naphtalènesulfonyloxy, soit non substitué soit substitué par un groupe alkyle en halogène et/ou nitro.

2. Composés selon revendication 1, dans lesquels
R est un groupe de formule Ia, Ib, Ie ou Id,
R₁ est un groupe 4-halogénobut-2-ényle,
R₂ est un groupe alkyle en C₁-C₇ ou cycloalkyle à 3 à 8 chaînons.
R₃ est un groupe alcoxy en C₁-C₇,
R₄ est un groupe (alcoxy en C₁-C₇)-alcoxy en C₁-C₇, ou si R est un groupe de formule (Ia), il s'agit d'un groupe hydroxy, hydroxy-alcoxy en C₁-C₇ ou d'un groupe de formule Ie,
R₅ est un halogène ou un groupe (alcane en C₁-C₇)-sulfonyloxy, halogène-(alcane en C₁-C₇)sulfonyle ; ou benzène-sulfonyloxy ou naphtalènesulfonyloxy qui est non substitué ou substitué par un groupe alkyle en C₁-C₇, un halogène et/ou un nitro,
R₆ est un groupe azido, et
R₇ est un groupe alkyle en C₁-C₇, alcényle en C₁-C₇, cycloalkyle à 3 à 8 chaînons, ou phényl-alkyle en C₁-C₇ qui est non substitué ou substitué dans la fraction phényle par un groupe alkyle en C₁-C₇, alcoxy en C₁-C₇, halogène et/ou nitro,
et leurs sels.

3. Composés selon la revendication 1, dans lesquels
R est un groupe de formule Ia, Ib, Ic ou Id,
R₁ est un groupe 4-halogénobut-2-ényle,
R₂ est un groupe alkyle en C₃-C₇ ramifié, tel que propyle,
R₃ est un groupe alcoxy en C₁-C₄, tel que methoxy,
R₄ est un groupe (alcoxy en C₁-C₄)-alcoxy en C₂-C₄, tel que 3-méthoxypropyloxy, ou si R est un groupe de formule (Ia), il s'agit d'un groupe hydroxy, hydroxy-alcoxy en C₂-C₄, tel que 3-hydroxypropyloxy, ou d'un groupe de formule Ie,
R₅ est un halogène de numéro atomique jusqu'à 35, 35 inclus, tel que brome,
R₆ est un groupe azido ou amino, et
R₇ est un groupe alkyle en C₃-C₇ ramifié, tel que propyle,
et leurs sels.

4. Composé selon la revendication 1, sélectionné parmi les suivants :
bis-(3-isovaléroyl-6-méthoxy-phenyl)-carbonate ;
1-(3-hydroxy-4-méthoxy-phényl)-3-methyl-butan-1-one ;
1-[3-(3-hydroxypropyloxy)-4-méthoxy-phényl]-3-méthyl-butan-1-one;
1-[4-méthoxy-3-(3-méthoxypropyloxy)-phényl]-3-méthyl-butan-1-one;
*trans*-1-bromo-6-[4-methoxy-3-(3-méthoxypropyloxy)-phényl]-5-(S)-isopropyl-hex-2-én-6-one;
3(S)-isopropyl-5(S)-{1(R)-bromo-3(S)-isopropyl-4-[4-méthoxy-3-(3-méthoxypropoxy)phényl]-4-oxo-butyl}-tetrahydrofurann-2-one ; et
3(S)-isopropyl-5(S)-{1(S)-azido-3(S)-isopropyl-4[4-méthoxy-3-(3-méthoxypropyloxy-phényl]-4-oxo-butyl}-tétrahydrofurann-2-one.

5. Procédé de production de composés de formule I 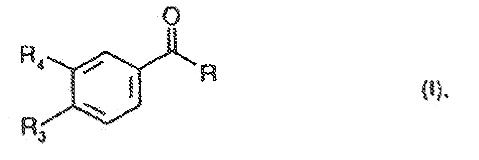 dans laquelle
R est un groupe de formule Ia, Ib, Ic ou Id 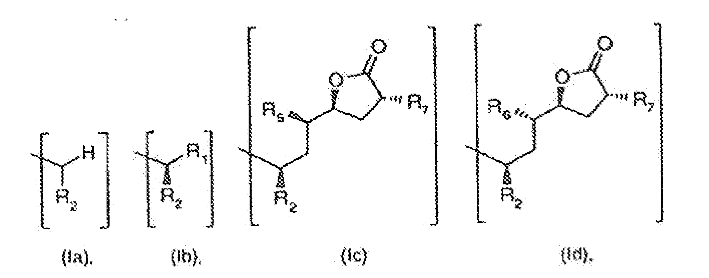 R₁ est un groupe 4-halogénobut-2-ényle,
R₂ est un groupe alkyle en C₁-C₇ ou cycloalkyle,
R₃ est un groupe alcoxy en C₁-C₇,
R₄ est un groupe (alcoxy en C₁₋C₇)-alcoxy en C₁-C₇, ou si R est un groupe de formule (Ia), il s'agit d'un groupe hydroxy, hydroxy-alcoxy en C₁-C₇ ou d'un groupe de formule Ie 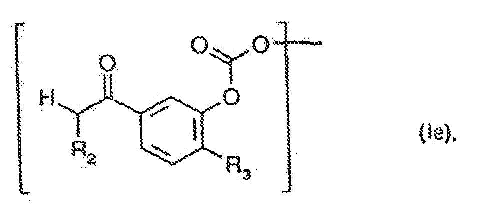 R₅ est un groupe hydroxyle estérifié réactif tel que défini dans Ia revendication 1,
R₆ est un groupe azido, et
R₇ est un groupe alkyle en C₁-C₇, alcényle en C₂-C₇, ou aryl-alkyle en C₁-C₇,
et leurs sels, **caractérisé en ce que**
a) pour produire des composés de formule I dans laquelle R est un groupe de formule Ia, R₂ est un groupe alkyle en C₁-C₇ ou cycloalkyle, R₃ est un groupe alcoxy en C₁-C₇ et R₄ est un groupe de formule Ie,
un composé de formule IV 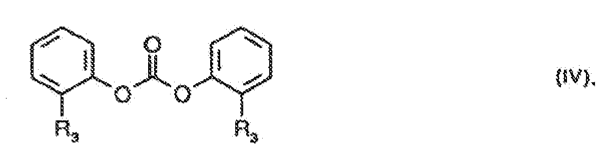 dans laquelle R₃ a la signification donnée précédemment, est condensé avec un composé de formule V 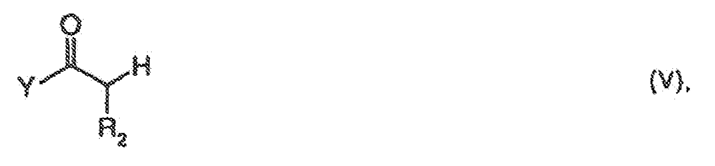 dans laquelle R₂ a la signification donnée précédemment et Y est un groupe hydroxy estérifié réactif,
b) pour produire un composé de formule I dans laquelle R est un groupe de formule Ia, R₂ est un groupe alkyle en C₁-C₇ ou cycloalkyle, R₃ est un groupe alcoxy en C₁-C₇ et R₄ est un groupe hydroxy, un composé de
formule VI 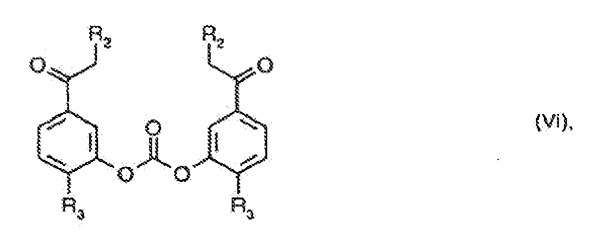 dans laquelle R₂ et R₃ ont les significations données précédemment, subit une solvolyse pour former les composés correspondants de formule I dans laquelle R₄ est un groupe hydroxy,
c) pour produire un composé de formule I dans laquelle R est un groupe de formule Ia, R₂ est un groupe alkyle en C₁-C₇ ou cycloalkyle, R₃ est un groupe alcoxy en C₁-C₇ et R₄ est un groupe hydroxy-alcoxy en C₁-C₇-R'₄, dans un composé de formule VII, 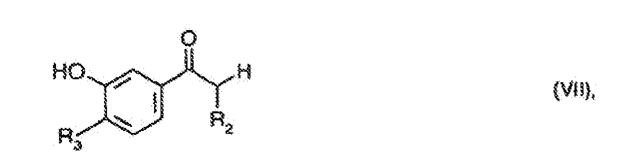 dans laquelle R₂ et R₃ ont les significations données précédemment, Ie groupe hydroxy est converti en groupe hydroxy-alcoxy en C₁-C₇-R'₄,
d) pour produire un composé de formule I dans laquelle R est un groupe de formule Ia, R₂ est un groupe alkyle en C₁-C₇ ou cycloalkyle, R₃ est un groupe alcoxy en C₁-C₇, R₄ est un groupe (alcoxy en C₁-C₇)-alcoxy en C₁-C₇-R"₄, dans un composé de formule VIII, 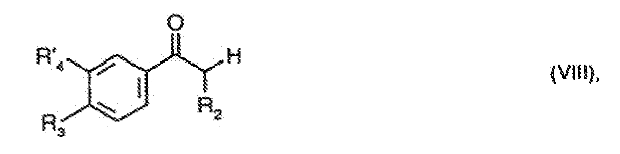 dans laquelle R₂ et R₃ ont les significations données précédemment, R'4 est un groupe hydroxy-alcoxy en C₁-C₇, le groupe hydroxy-alcoxy en C₁-C₇ est converti en groupe (alcoxy en C₁-C₇)-alcoxy en C₁-C₇-R"4,
e) pour produire des composés de formule I dans laquelle R est un groupe de formule Ib, R₁ est un groupe 4-halogene-but-2-ényle, R₂ est un groupe alkyle en C₁-C₇, ou cycloalkyle, R₃ est un groupe alcoxy en C₁-C₇ et R₄ est un groupe (alcoxy en C₁-C₇)-alcoxy en C₁-C₇-R"₄, un composé de formule IX, 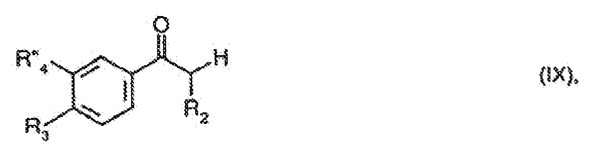 dans laquelle R₂, R₃ et R"₄ ont les significations données précédemment, est mis à réagir d'abord avec une amine primaire chirale qui est usuelle à des fins de synthèse stéréosélective, puis avec un 1-4-dihalogène-but-2-ène,
f) pour produire des composés de formule I dans laquelle R est un groupe de formule Ic, R₂ est un groupe alkyle en C₁-C₇ ou cycloalkyle, R₃ est un groupe alcoxy en C₁-C₇, R₄ est un groupe (alcoxy en C₁-C₇)-alcoxy en C₁-C₇-R"4, R₅ est un groupe hydroxy estérifié réactif et R₇ est un groupe alkyle en C₁-C₇, alcényle en C₁-C₇, cycloalkyle ou aryl-alkyle en C₁-C₇,
un composé de formule X, 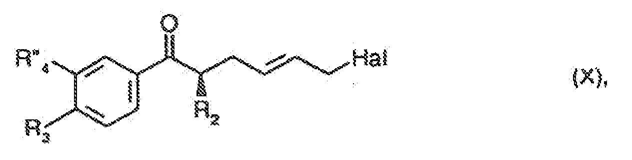 dans laquelle R₂, R₃ est R"₄ ont les significations données précédemment, et HaI est une halogène, est mis à réagir avec un composé de formule XI 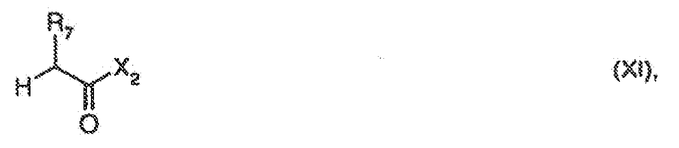 dans laquelle R₇ a la signification donnée précédemment et X₂ est un groupe amino, amido ou uréthane chiral qui est usuel à des fins de synthèse stéréosélective, ou est un groupe alcool chiral ; ou un 1-4-dihalogène-but-2-ène est mis à réagir d'abord avec une composé de formule XI puis avec le produit de réaction de la réaction d'un composé de formule IX 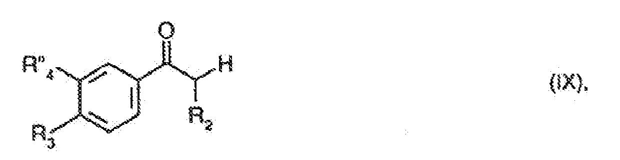 avec une amine primaire chirale de formule XII qui est usuelle à des fins de synthèse stéréosélective 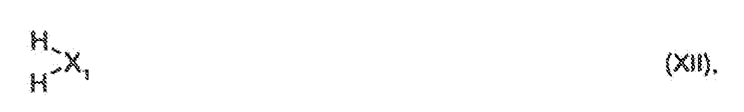 et le produit de réaction de formule XIII 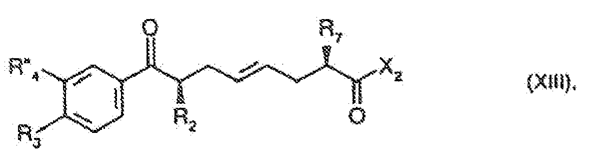 est condensé de façon intramoléculaire avec lactonisation pour former les composés correspondants de formule I, dans laquelle R est un groupe de formule Ic et R₅ est un halogène, et si désiré, l'halogène R₅ est converti de façon stéréosélective en un autre groupe hydroxy éthérifié réactif R₅, et/ou g) pour produire des composés de formule I dans laquelle R est un groupe de formule Id, R₂ est un groupe alkyle en C₁-C₇ ou cycloalkyle, R₃ est un groupe alcoxy en C₁-C₇, R₄ est un groupe (alcoxy en C₁-C₇)-alcoxy en C₁-C₇-R''₄, R₆ est un groupe azido et R₇ est un groupe alkyle en C₁-C₇, alcényle en C₁-C₇, cycloalkyle ou aryl-alkyle en C₁-C₇, dans un composé de formule XIV 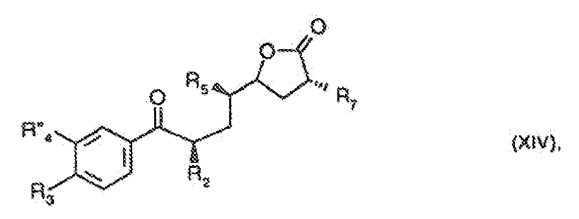 dans laquelle R₂, R₃, R"₄ et R₇ ont les significations données précédemment, et R₅ est un groupe hydroxyle estérifié réactif tel que défini dans la revendication 1, le groupe hydroxy estérifié réactif R₅ est remplacé de façon stéréospécifique par un groupe azido, et si désiré, un composé libre pouvant être obtenu selon le procédé est converti en un sel, ou un sel pouvant être obtenu selon le procédé est converti en composé libre ou en un autre sel.

6. Procédé de production de composés de formule II 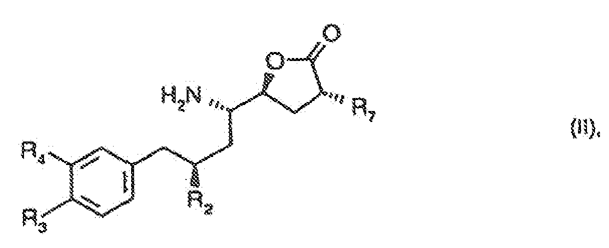 dans laquelle R₂ est un groupe alkyle en C₁-C₇ ou cycloalkyle,
R₃ est un groupe alcoxy en C₁-C₇,
R₄ est un groupe (alcoxy en C₁-C₇)-alcoxy en C₁-C₇ et
R₇ est un groupe alkyle en C₁-C₇, alcényle en C₁-C₇, cycloalkyle ou aryl-alkyle en C₁-C₇,
**caractérisé en ce que**
a) un composé de formule IV 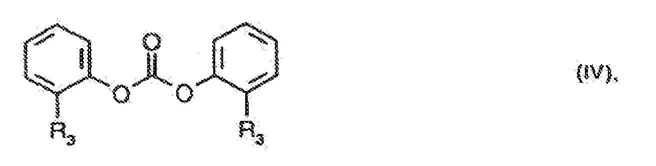 dans laquelle R₃ a la signification donnée précédemment, est condensé avec un composé de formule V 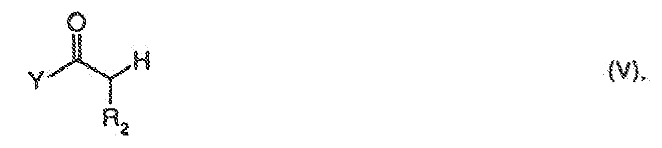 dans laquelle R₂ a la signification donnée précédemment et Y est un groupe hydroxy estérifié réactif,
b) dans le composé obtenu de formule VI 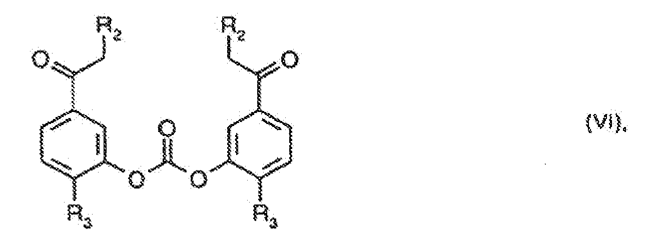 dans laquelle R₂ et R₃ ont les significations données précédemment, subit une solvolyse pour former les composés correspondants de formule I dans laquelle R₄ est un groupe hydroxy,
c) dans les composés obtenus de formule VII, 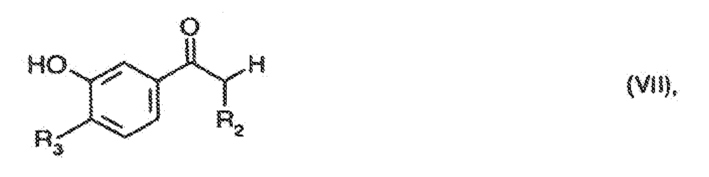 dans laquelle R₂ et R₃ ont les significations données précédemment, le groupe hydroxy est converti en groupe hydroxy-alcoxy en C₁-C₇-R'₄,
d) dans les composés obtenus de formule VIII, 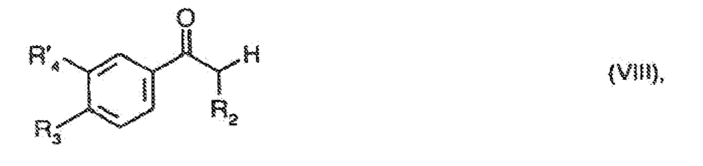 dans laquelle R₂ et R₃ ont les significations données précédemment, R'₄ est un groupe hydroxy-alcoxy en C₁-C₇, le groupe hydroxy-alcoxy en C₁-C₇ est converti en groupe (alcoxy en C₁-C₇)-alcoxy en C₁-C₇-R"₄,
e) le composé obtenu de formule IX, 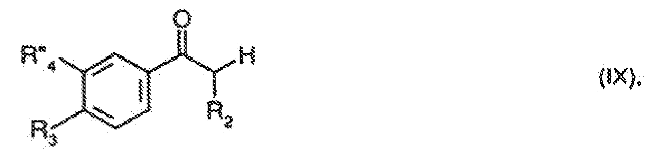 dans laquelle R₂, R₃ et R"₄ ont les significations données précédemment, est mis à réagir d'abord avec une aminé primaire chirale qui est usuelle à des fins de synthèse stéreoselective, puis avec un 1-4-dihalogène-but-2-ène,
f) le composé obtenu de formule X, 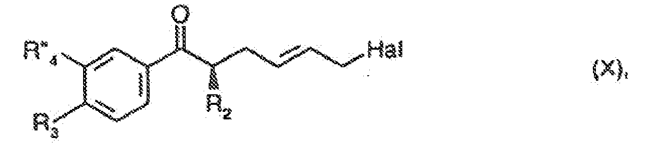 dans laquelle R₂, R₃ et R"₄ ont les significations données précédemment, est Hal est un halogène, est mis à réagir avec un composé de formule XI 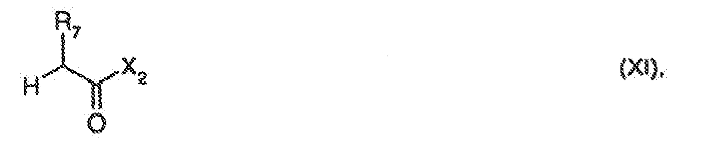 dans laquelle R₇ a la signification donnée précédemment et X₂ est un groupe amino, amido ou uréthane chiral qui est usuel à des fins de synthèse stéréosélective, ou est un groupe alcool chiral; ou un 1-4-dihalogéne-but-2-ène est mis à réagir d'abord avec un composé de formule XI, puis avec le produit de réaction de la réaction d'un composé de formule IX 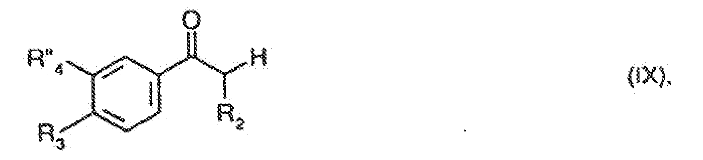 avec une amine primaire chirale de formule XII qui est usuelle à des fins de synthèse steréosélective, 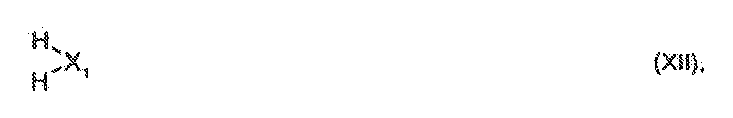 et le produit de réaction de formule XIII 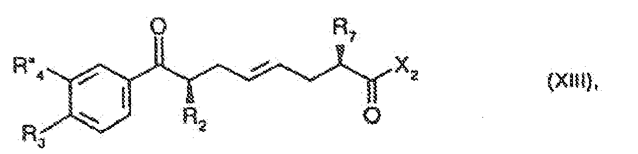 est condensé de façon intramoléculaire avec lactonisation pour former les composés correspondants de formule I, dans laquelle R est un groupe de formule Ic et R₅ est un halogène, et si désiré, l'halogène est converti de façon stéréosélective en un autre groupe hydroxy éthérifié réactif R₅ tel que défini dans la revendication 1,
g) dans les composés obtenus de formule XIV 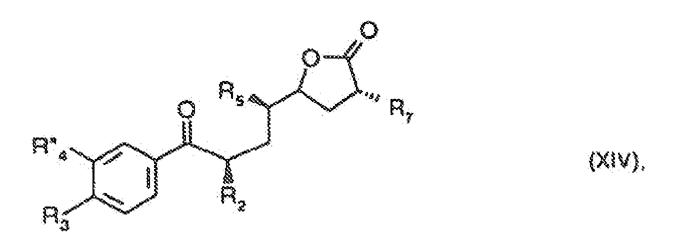 dans laquelle R₂, R₃, R"₄ et R₇ ont les significations données précédemment, et R₅ est un groupe hydroxyle estérifié réactif tel que défini dans la revendication 1, le groupe hydroxy estérifié réactif R₅ est remplacé de façon stéréospécifique par un groupe azido, et
h) dans un composé de formule I, dans laquelle R est un groupe de formule Id, R₂ est un groupe alkyle en C₁-C₇ ou cycloalkyle, R₃ est un groupe alcoxy en C₁-C₇, R₄ est un groupe (alcoxy en C₁-C₇)-alcoxy en C₁-C₇, R₆ est un groupe azido et R₇ est un groupe alkyle en C₁-C₇, alcényle en C₁-C₇, cycloalkyle ou aryl-alkyle en C₁-C₇, le groupe azido R₆ est réduit en un groupe amino, et dans le même temps ou ultérieurement, le groupe benzoyle est réduit en groupe benzyle correspondant, et si désiré, un composé libre pouvant être obtenu selon le procédé est converti en un sel, ou un sel pouvant être obtenu selon le procédé est converti en composé libre ou en un autre sel.
